# EUROPÄISCHE PATENTSCHRIFT

(11) **EP 3 675 942 B1**
(45) Veröffentlichungstag und Bekanntmachung des Hinweises auf die Patenterteilung: **27.09.2023**
(21) Anmeldenummer: 19711313.7
(22) Anmeldetag: 13.03.2019
(51) Int. Cl.: A61M 16/00, A61G 12/00, A61M 16/08

(54) **BEATMUNGSGERÄT MIT VERSTELLBARER BENUTZERSCHNITTSTELLE**
MECHANICAL VENTILATOR HAVING AN ADJUSTABLE USER INTERFACE
APPAREIL DE VENTILATION AYANT UNE INTERFACE UTILISATEUR RÉGLABLE

(30) Priorität: 16.03.2018 DE 102018002137; 04.07.2018 DE 102018005280
(43) Veröffentlichungstag der Anmeldung: 08.07.2020
(73) Patentinhaber: Drägerwerk AG & Co. KGaA, 23558 Lübeck (DE)
(72) Erfinder: DASKE, Jochen, 23552 Lübeck (DE); HAMPE, Markus, 23562 Lübeck (DE); LISCHINSKI, Robert, 23911 Einhaus (DE); NANDZIK, Andreas, 12309 Berlin (DE); ENGELSMAN, Eduard, 23552 Lübeck (DE); ECKERMANN-BRAHE, Thomas, 46485 Wesel (DE); PAUL, Christof, 48165 Münster (DE); SAVCHUK, Volodymyr, 23617 Stockelsdorf (DE)
(86) Internationale Anmeldenummer: PCT/EP2019/056253
(87) Internationale Veröffentlichungsnummer: WO 2019/175221

(56) Entgegenhaltungen:
- EP-A1- 1 396 235
- EP-A1- 1 396 235
- WO-A1-2008/010004
- WO-A1-2016/043644
- WO-A2-2012/158560
- CN-A- 105 411 557
- US-A1- 2008 000 477
- US-A1- 2015 202 105
- US-B1- 6 339 732
- US-B2- 8 011 362
- US-B2- 8 920 362

## Beschreibung

Die Erfindung betrifft ein Beatmungsgerät für den klinischen Einsatz, bestehend aus einem Grundgerät, einer zugehörigen Benutzerschnittstelle und einem optionalen Fahrgestell, auf dem das Beatmungsgerät angebracht ist.

### STAND DER TECHNIK

Ein Beatmungsgerät weist üblicherweise ein quaderförmiges Gehäuse zur Aufnahme von pneumatischen und elektronischen Komponenten auf und besitzt Schnittstellen für Gasein- und -ausgänge sowie weitere, hauptsächlich elektrische Schnittstellen. Von besonderer Bedeutung ist dabei die Anordnung der Patientenanschlüsse für die Beatmungsschläuche, die zum Patienten gehen bzw. vom Patienten zurück zum Gerät.

Moderne Beatmungsgeräte werden über eine großflächige Benutzerschnittstelle mit einem Touchscreen gesteuert, der wichtige Beatmungsparameter, wie zum Beispiel eingestellte und gemessene Therapiewerte und Druck- und Volumenkurven, anzeigt und dem Anwender vielfältige Interaktionen zur Verfügung stellt. Häufig sind unterhalb oder neben dem Touchscreen weitere Hardware-Bedienelemente für spezielle Funktionen, wie ein Drehknopf und/oder Tasten angeordnet.

Beatmungsgeräte in unterschiedlichen Leistungsklassen unterscheiden sich hinsichtlich der Gehäusekonstruktion häufig durch die Art, wie die Benutzerschnittstelle mit dem Grundgerät verbunden ist.

Geräte der unteren Leistungsklassen integrieren die oft vergleichsweise kleine Benutzerschnittstelle in das Grundgerät, vielfach in einem nach ergonomischen Kriterien ausgelegten, aber nicht veränderbaren Winkel. Benutzerschnittstelle und Patientenanschlüsse sind bei solchen Geräten meistens in dieselbe Richtung orientiert. Vorteile einer solchen Ausführungsform sind die geringen Geräteabmessungen und der kostengünstige Gehäuseaufbau.

Geräte höherer Leistungsklassen haben dagegen häufig eine vom Grundgerät abgesetzte Benutzerschnittstelle, die beispielsweise über eine senkrechtstehende Achse und ein Schwenkgelenk gegenüber dem Grundgerät in zwei Freiheitsgraden frei positionierbar ist. Die Benutzerschnittstelle ist dann durch ein offenliegendes Kabel, insbesondere ein Systemkabel, mit dem Grundgerät verbunden. Häufig besteht bei solchen Geräten auch die Möglichkeit, die Benutzerschnittstelle abzunehmen und an anderer Stelle in der Nähe des Patienten anzubringen, zum Beispiel mittels einer Schienenklaue an einer Normschiene. Der Vorteil einer solchen flexiblen Anordnung ist, dass sowohl Patientenanschlüsse als auch Benutzerschnittstelle jeweils ergonomisch optimal positioniert werden können, sodass zum Beispiel Patientenanschlüsse in Richtung des Patienten und die Benutzerschnittstelle in Richtung Arzt positioniert werden können.

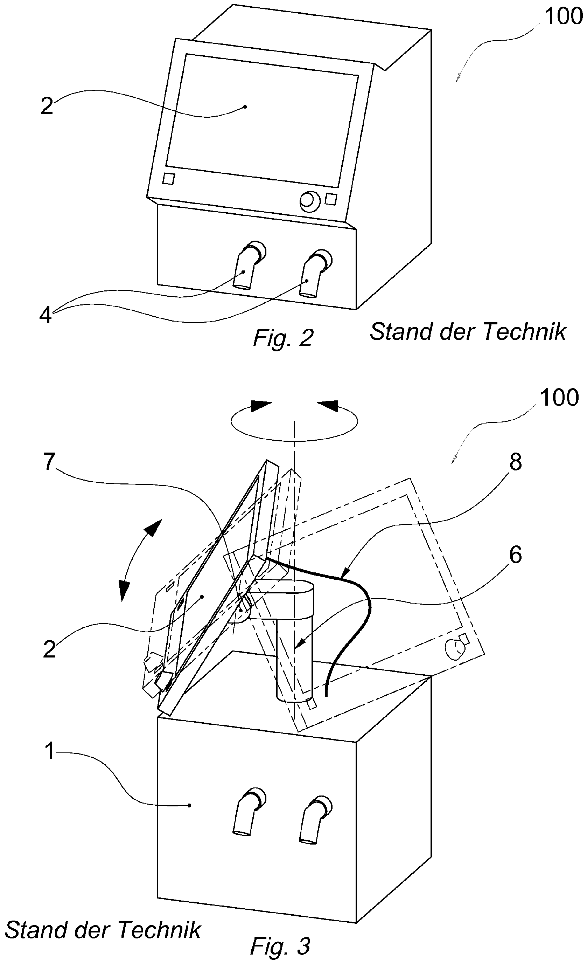

Den Stand der Technik auf Seiten der kompakten Beatmungsgeräte stellen im Wesentlichen Geräte mit einer Gehäusearchitektur, wie sie beispielsweise in Fig. 2 gezeigt wird, dar. Ein derartiges Gerät ist beispielsweise die Dräger Savina 300^{®} der Drägerwerk AG & Co. KGaA. Andere Geräte weisen dagegen eine halbintegrierte Benutzerschnittstelle auf, die mittels eines Schwenkgelenks in der Neigung einstellbar ist. Ein Beispiel für ein derart ausgeführtes Gerät ist das Beatmungsgerät SV 300 der Firma Mindray.

Die zuvor beschriebenen technischen Lösungen zeichnen sich dadurch aus, dass die relative Position zwischen Benutzerschnittstelle und Patientenanschlüssen nicht veränderbar ist. Sofern die Patientenanschlüsse zur Einsparung von Länge der Beatmungsschläuche, was im Hinblick auf eine Reduktion des Totraums und damit für eine verbesserte Beatmungsperformance wünschenswert ist, in Richtung des Patienten ausgerichtet werden, ist das Display nicht optimal zum Arzt ausgerichtet. Außerdem ist eine ergonomisch optimierte Neigungseinstellung der Benutzerschnittstelle zumindest bei den Geräten der zuvor beschriebenen Bauarten ohne Neigungseinstellung nicht möglich. Weiterhin stellt es vielfach ein Problem dar, dass aufgrund der Verwendung eines großflächigen Touchscreens, wie für moderne Graphical User Interfaces gefordert, es zwangsläufig zu einer Vergrößerung der Bauhöhe des gesamten Gerätes führt, da die Benutzerschnittstelle oberhalb der Patientenanschlüsse angeordnet ist.

Neben den zuvor beschriebenen Beatmungsgeräten sind Geräte höherer Leistungsklassen bekannt, die über ein separates Bedienteil verfügen. Die Gehäusearchitektur dieser Geräte ist in Fig. 3 dargestellt. Ein Beispiel für ein derartiges Gerät stellt die Dräger Evita^{®} Infinity^{®} 500 der Drägerwerk AG & Co. KGaA dar. Ferner ist das Gerät Servo-i^{®} der Firma Maquet bekannt, das durch Umdrehen des Grundgeräts auf dem Trolley um 180° zusätzlich ein Umkonfigurieren der Patientenanschlüsse von einer auf die andere Seite ermöglicht.

Die zuvor beschriebenen technischen Lösungen zeichnen sich durch einen hohen Aufbau aufgrund der Anordnung der Benutzerschnittstelle über dem Grundgerät aus, sodass in der Vertikalen viel Bauraum benötigt wird. Bei diesen Geräten ist daher entweder ein Systemverbund, der den Trolley als Tragstruktur mit einschließt, oder eine separate Montage von Grundgerät und Benutzerschnittstelle an stationären Haltesystemen üblich. Aus diesem Grund ist der konstruktive Aufwand vergleichsweise hoch und Achse, Schwenkgelenk sowie Kabel bzw. Systemkabel liegen offen und bilden zusätzliche Außenflächen, die bei der Aufbereitung des Geräts mit desinfiziert werden müssen.

Beatmungsgeräte mit einer beweglichen Benutzerschnittstelle sind aus der US 2008/0000477 A1, US 2015/0202105 A1, US 8 011 362 B2 und WO 2016/043644 A1 bekannt. Geräte mit einem Grundgerät, einer relativ zum Grundgerät verdrehbar gekoppelten Platte und einer mit der Platte beweglich gekoppelten Benutzerschnittstelle sind aus der US 6 339 732 B1 und EP 1 396 235 A1 bekannt.

### OFFENBARUNG DER ERFINDUNG

Ausgehend von den aus dem Stand der Technik bekannten technischen Lösungen liegt der Erfindung die Aufgabe zugrunde, ein Beatmungsgerät bereitzustellen, das die vorteilhaften Merkmale eines Kompaktgeräts, wie es in Fig. 2 gezeigt ist, mit denen eines vergleichsweise flexiblen Geräts höherer Leistungsklasse, wie es in Figur 3 zu sehen ist, vereint. Insbesondere soll ein Gerät angegeben werden, das flexibel einsetzbar ist und gleichzeitig über einen vergleichsweise einfachen Aufbau und leicht zu bedienenden Mechanismus zur Bewegung einer Benutzerschnittstelle, wie z.B. eines Displays und/oder einer Bedieneinheit, verfügt. Dabei ist es bevorzugt, dass die Benutzerschnittstelle des Geräts einfach und sicher in unterschiedliche Positionen überführt werden kann, sodass die Patientenschlüsse für die erforderlichen Beatmungsschläuche auf unterschiedlichen Seiten relativ zum Display und/oder zur Bedieneinheit angeordnet sind. Ferner soll sich das anzugebende Beatmungsgerät durch eine einfache Gestaltung mit möglichst geringen Außenabmessungen auszeichnen, dennoch aber mit einem möglichst großen Bedienteil versehbar sein. Insbesondere soll ein Gerät bereitgestellt werden, bei dem mit wenigen Handgriffen eine Anordnung, bei der die Patientenanschlüsse relativ zum Bedienteil und/oder Display nach links zeigen, in eine entgegengesetzte Anordnung mit nach rechts zeigenden Patientenanschlüssen umkonfiguriert werden kann. Die optionale Einstellbarkeit einer dritten Konfiguration mit nach vorne ausgerichteten Patientenanschlüssen wäre weiterhin vorteilhaft.

Voranstehende Aufgabe wird durch die Patentansprüche gelöst. Demnach wird die Aufgabe durch ein Beatmungsgerät mit den Merkmalen des unabhängigen Anspruchs 1 gelöst. Weitere Merkmale und Details der Erfindung ergeben sich aus den Unteransprüchen, der Beschreibung und den Zeichnungen.

Das erfindungsgemäße Beatmungsgerät weist ein Grundgerät mit einer Deckplatte, am Grundgerät angeordnete Patientenanschlüsse und eine an der Deckplatte angeordnete Benutzerschnittstelle auf. Erfindungsgemäß ist die Deckplatte relativ zum Grundgerät um unterschiedliche Winkel in Bezug auf eine vertikale Achse an dem Grundgerät anordenbar. Das Beatmungsgerät kann erfindungsgemäß auch als integraler Bestandteil eines Anästhesiegeräts zur Narkotisierung eines Patienten ausgebildet sein.

Das Grundgerät des Beatmungsgeräts weist vorzugsweise ein Gehäuse auf, welches einen Innenraum von einer Umgebung abschirmt. Zudem ist es bevorzugt, dass das Grundgerät als Kerngerät des Beatmungsgeräts und somit zum Erzeugen eines Atemluftstroms ausgebildet ist. Vorzugsweise ist an dem Grundgerät eine Anschlussvorrichtung für eine Stromversorgung angeordnet. Ferner ist das Grundgerät über eine elektrische Verbindung mit der Benutzerschnittstelle elektrisch gekoppelt, sodass technische Funktionen des Grundgeräts mittels der Benutzerschnittstelle steuerbar sind. Die Patientenanschlüsse sind vorzugsweise seitlich an dem Grundgerät angeordnet. Es kann auch vorgesehen sein, dass die Patientenanschlüsse an einer Unterseite des Grundgeräts angeordnet sind. Alternativ können die Patientenanschlüsse über mehrere Seiten des Grundgeräts verteilt sein.

Die Deckplatte weist vorzugsweise eine Form auf, welche einer Grundrissform des Grundgeräts entspricht, sodass die Deckplatte zumindest bei entsprechender Ausrichtung vorzugsweise bündig mit Seitenwänden des Grundgeräts abschließt. Die Deckplatte ist um die vertikale Achse um unterschiedliche Winkel relativ zum Grundgerät verdrehbar.

Die Benutzerschnittstelle ist vorzugsweise als Display und/oder Bedienteil ausgebildet. Eine bevorzugte Benutzerschnittstelle ist als Touchscreen-Monitor ausgebildet. Zusätzlich oder alternativ kann die Benutzerschnittstelle mechanische Tasten und/oder Drehgeber aufweisen. Zudem ist die Benutzerschnittstelle zumindest einem Abschnitt der Deckplatte benachbart angeordnet. Durch ein relatives Verstellen eines Winkels der Deckplatte zum Grundgerät in Bezug auf die vertikale Achse ist somit die Benutzerschnittstelle relativ zum Grundgerät in Bezug auf die vertikale Achse in eine andere Position bewegbar. Vorzugsweise ist die Benutzerschnittstelle derart an der Deckplatte anordenbar, dass diese schräg zur Deckplatte angeordnet ist. Dabei ist es bevorzugt, wenn eine die Deckplatte enthaltende Ebene die Benutzerschnittstelle entlang einer Geraden schneidet. Es ist besonders bevorzugt, dass die Benutzerschnittstelle an einem Randbereich der Deckplatte gehalten ist. Weiter bevorzugt ist ein Randbereich der Benutzerschnittstelle an der Deckplatte gehalten.

Das erfindungsgemäße Beatmungsgerät hat gegenüber herkömmlichen Beatmungsgeräten den Vorteil, dass bei einer besonders kompakten Baugröße ein Anordnen der Benutzerschnittstelle in unterschiedlichen Positionen relativ zum Grundkörper gewährleistet ist. Die Benutzerschnittstelle ist insbesondere relativ zu einem Fahrgestell des Beatmungsgeräts und/oder einer Funktionseinheit des Beatmungsgeräts mit Ventilator und den erforderlichen pneumatischen Bauteilen anordenbar. Auf diese Weise sind die Patientenschlüsse für die Beatmungsschläuche je nach Anordnung des Bedienteils, Monitors und/oder Displays an unterschiedlichen Seiten des Bedienteils, Monitors und/oder Displays anordenbar. Somit ist eine variable relative Positionierung von Benutzerschnittstelle zu den Patientenschlüssen gewährleistet. Das erfindungsgemäße Beatmungsgerät ist demnach auf eine besonders vorteilhafte Art und Weise für unterschiedliche Anwendungsfälle umkonfigurierbar.

Vorzugsweise ist die Deckplatte über einen Drehmechanismus um eine vertikale Achse verdrehbar an dem Grundgerät gehalten. Aus diesem Grund ist die Deckplatte vorzugsweise unter Ausbildung eines kleinen Spalts am Grundgerät angeordnet, um ein Schleifen zwischen Deckplatte und Grundgerät zu vermeiden. Dabei ist die Deckplatte möglichst nah an dem Grundgerät angeordnet, um eine besonders kompakte Baugröße des Beatmungsgeräts sicherzustellen. Der Drehmechanismus weist beispielsweise eine Wälzlagerung und/oder eine Gleitlagerung oder dergleichen auf. Ein Drehmechanismus hat den Vorteil, dass die Anordnung der Deckplatte zum Grundgerät um unterschiedlichen Winkel in Bezug auf die vertikale Achse verbessert ist, da die Relativbewegung der Deckplatte zum Grundgerät über den Drehmechanismus geführt ist und somit als Rotation erfolgen kann.

Es kann erfindungsgemäß vorgesehen sein, dass das Beatmungsgerät, insbesondere der Drehmechanismus, einen Rastmechanismus aufweist, welcher ein relatives Verdrehen der Deckplatte zum Grundgerät in vordefinierten Abständen, insbesondere in 90° oder 180° Abständen, spürbar erschwert. Es können beispielsweise Rastpositionen bei 0°, 90°, 180° und 270° vorgesehen sein. Vorzugsweise entsprechen die Rastpositionen Gebrauchspositionen der Deckplatte. Derartige Rastpositionen haben den Vorteil, dass ein definiertes relatives Ausrichten von Deckplatte zum Grundgerät erleichtert ist.

Vorzugsweise weist das Beatmungsgerät Drehanschläge zum Begrenzen einer Drehung der Deckplatte um die vertikale Achse relativ zum Grundgerät auf. Die Drehanschläge können beispielsweise an dem Drehmechanismus ausgebildet sein. Alternativ oder zusätzlich können die Drehanschläge an dem Grundgerät sowie der Deckplatte angeordnet sein. Vorzugsweise sind die Drehanschläge derart ausgebildet und angeordnet, dass ein relatives Verdrehen der Deckplatte zum Grundgerät von etwa 360°, insbesondere von 180°, möglich ist. Durch eine Begrenzung der Drehbewegung der Deckplatte wird ein unkontrolliertes Verdrillen von Kabeln, welche das Grundgerät mit dem Bedienteil verbinden, auf vorteilhafte Weise sowie mit einfachen Mitteln verhindert.

Gemäß einer bevorzugten Weiterbildung der Erfindung kann bei einem erfindungsgemäßen Atemgerät vorgesehen sein, dass die Deckplatte einen um 180° oder um 90° rotationssymmetrisch ausgebildeten Grundriss aufweist. Unter einem rotationssymmetrisch ausgebildeten Grundriss wird im Rahmen der Erfindung auch ein im Wesentlichen rotationssymmetrisch ausgebildeter Grundriss verstanden, sodass die Deckplatte beispielsweise eine lokale Ausnehmung bzw. Freimachung und/oder Formzugabe aufweisen kann, durch welche eine reine Rotationssymmetrie zerstört würde. Eine um 180° rotationssymmetrisch ausgebildete Deckplatte weist beispielsweise eine rechteckige oder eine ovale Grundfläche auf. Eine um 90° rotationssymmetrisch ausgebildete Deckplatte weist beispielsweise eine quadratische oder eine runde Grundfläche auf. Dabei ist es bevorzugt, dass die Grundplatte fluchtend oder zumindest symmetrisch zu Seitenwänden des Grundgeräts anordenbar ist. Eine derartige, insbesondere rechteckige oder quadratische, Deckplatte hat den Vorteil, dass eine lagegetreue Ausrichtung der Deckplatte zum Grundgerät leicht erzielbar ist. Somit ist mittels des erfindungsgemäßen Beatmungsgeräts eine vordefinierte relative Lage von Benutzerschnittstelle und den Patientenanschlüssen mit einfachen Mitteln einstellbar.

Erfindungsgemäß ist die Benutzerschnittstelle über ein Schwenkgelenk um eine horizontale Schwenkachse verschwenkbar an der Deckplatte gehalten. Das Schwenkgelenk kann beispielsweise als Scharniergelenk, Kugelgelenk oder dergleichen ausgebildet sein. Mittels des Schwenkgelenks ist die Benutzerschnittstelle drehbeweglich an der Deckplatte gelagert. Vorzugsweise ist das Schwenkgelenk an einem Randbereich der Benutzerschnittstelle und/oder einem Randbereich der Deckplatte angeordnet bzw. anordenbar. Auf diese Weise ist ein relativer Winkel der Benutzerschnittstelle zur Deckplatte und somit auch zum Grundgerät einstellbar. Ein Schwenkgelenk hat den Vorteil, dass eine Ausrichtung der Benutzerschnittstelle zu einem Bediener des Beatmungsgeräts, wie zum Beispiel einem Arzt, mit einfachen Mitteln sowie kostengünstig verbesserbar ist.

Es ist ferner bevorzugt, dass das Beatmungsgerät eine Rastvorrichtung zum Halten der Benutzerschnittstelle in einer Rastposition relativ zur Deckplatte aufweist. Besonders bevorzugt ist die Rastvorrichtung an dem Schwenkgelenk angeordnet bzw. ausgebildet. Vorzugsweise ist die Rastvorrichtung zum Halten der Benutzerschnittstelle in mehreren Rastpositionen ausgebildet. Auf diese Weise ist ein eingestellter relativer Winkel der Benutzerschnittstelle zur Deckplatte sowie zum Grundgerät temporär fixierbar. Ein selbsttätiges Verstellen des Winkels ist somit mit einfachen Mitteln sowie auf eine kostengünstige Art und Weise vermeidbar.

Gemäß einer bevorzugten Ausführungsform der Erfindung ist das Schwenkgelenk ausgebildet, die Benutzerschnittstelle in eine zumindest annähernd horizontale Position zu verschwenken. Eine horizontale Position bedeutet im Rahmen der Erfindung, dass die Benutzerschnittstelle parallel oder zumindest im Wesentlichen parallel zur Deckplatte angeordnet ist. Dies hat insbesondere von Vorteil, dass eine Kollision der Benutzerschnittstelle mit dem Grundgerät beim relativen Verdrehen der Deckplatte zum Grundgerät auf diese Weise besser vermeidbar ist. Zudem ist eine derartige Verschwenkbarkeit vorteilhaft wenn das Schwenkgelenk an einem Randbereich der Benutzerschnittstelle und/oder einem Randbereich des Grundgeräts angeordnet bzw. anordenbar ist. In diesem Fall ist die Benutzerschnittstelle beispielsweise derart verschwenkbar, dass diese die Deckplatte verdeckt bzw. innerhalb einer Grundrissform der Deckplatte angeordnet ist. Eine derartige Schwenkbarkeit hat den Vorteil, dass äußere Abmessungen des Beatmungsgeräts somit reduzierbar sind.

Erfindungsgemäß ist die Benutzerschnittstelle in einer Gebrauchsposition der Deckplatte mittels des Schwenkgelenks in eine Sperrposition verschwenkbar, wobei ein relatives Verdrehen der Deckplatte zum Grundgerät in der Sperrposition gesperrt ist. Demnach ist die Benutzerschnittstelle in eine Freigabeposition verschwenkbar, in welcher ein relatives Verdrehen der Deckplatte zum Grundgerät freigegeben ist. Eine Sperrposition ist vorzugsweise derart festgelegt, dass die Benutzerschnittstelle in einer Bedienposition verschwenkt ist. Die Bedienposition kann sich beispielsweise über einen Verschwenkbereich, insbesondere zwischen einer vertikalen Ausrichtung der Benutzerschnittstelle bis zu einer Übergangsausrichtung der Benutzerschnittstelle erstrecken. Die Freigabeposition ist vorzugsweise derart definiert, dass die Benutzerschnittstelle zwischen der Übergangsausrichtung und der horizontalen Ausrichtung verschwenkt ist. Die Übergangsausrichtung weicht vorzugsweise um zwischen 0° und 20° von der horizontalen Ausrichtung ab. Das Sperren in der Sperrposition erfolgt durch die Benutzerschnittstelle, welche in der Sperrposition mit dem Grundgerät formschlüssig in Eingriff gerät. Eine Verschwenkbarkeit in eine Sperrposition hat den Vorteil, dass ein unbeabsichtigtes Verdrehen der Deckplatte aus einer Gebrauchsposition bzw. einer Rastposition mit einfachen Mitteln sowie auf eine kostengünstige Art und Weise vermeidbar ist.

Es ist erfindungsgemäß bevorzugt, dass das Schwenkgelenk ausgebildet ist, in der Sperrposition die Deckplatte in einer Gebrauchsposition zu sperren. Vorzugsweise ist das Schwenkgelenk ausgebildet, in der Sperrposition die Deckplatte in mehreren verschiedenen vorgesehenen Gebrauchspositionen zu sperren. Demnach weist das Schwenkgelenk eine Ausbildung auf, welche die Sperrung bewirkt, wenn das Schwenkgelenk in die Sperrposition verschwenkt ist. Dabei ist das Schwenkgelenk vorzugsweise ausgebildet, in der Sperrposition mit dem Grundgerät, insbesondere formschlüssig, in Eingriff zu stehen. Eine derartige Ausbildung des Schwenkgelenks hat den Vorteil, dass eine Beschädigung der Benutzerschnittstelle mit einfachen Mitteln sowie auf eine kostengünstige Art und Weise vermeidbar ist.

Vorzugsweise ist das Grundgerät auf einem Trolley des Beatmungsgeräts angeordnet. Ein Trolley ist im Rahmen der Erfindung eine Vorrichtung, welche eine

Verschiebbarkeit des Beatmungsgeräts auf einer Bodenfläche begünstigt. Vorzugsweise weist der Trolley hierfür Räder und/oder Rollen auf. Weiter bevorzugt weist der Trolley eine Stativvorrichtung auf, um eine Anordnung des Grundgeräts auf einer Arbeitshöhe, beispielsweise 100 cm, zu ermöglichen. Die Stativvorrichtung ist vorzugsweise teleskopierbar ausgebildet, um die Anordnung des Grundgeräts auf verschiedenen Arbeitshöhen zu ermöglichen. Zudem kann bei dem Trolley vorgesehen sein, dass dieser Haltevorrichtungen für Gasflaschen, Anfeuchter, Gelenkarme oder dergleichen aufweist. Ein Trolley hat den Vorteil, dass eine Positionierung des Beatmungsgeräts, insbesondere der Benutzerschnittstelle sowie der Patientenanschlüsse, mit einfachen Mitteln sowie kostengünstig verbessert ist und ein Beatmungsgerät je nach Bedarf einfach von einem Aufbewahrungsraum zum Patienten und/oder von Patient zu Patient bewegt werden kann. Ein Trolley ermöglicht zudem den Transport eines beatmungspflichtigen Patienten z. B. zu einer Diagnoseeinrichtung, indem Patientenbett und Trolley mit Beatmungsgerät gemeinsam bewegt werden.

Weiter bevorzugt ist das Grundgerät über eine Aufnahmeplatte des Trolleys an dem Trolley lösbar gehalten. Die Aufnahmeplatte weist hierfür Aufnahmen auf, welche mit Gegenaufnahmen des Grundgeräts zusammenführbar sind. Die Aufnahmen können beispielsweise als Ausnehmungen und die Gegenaufnahmen als Zapfen ausgebildet sein. Die Aufnahmeplatte ist vorzugsweise um 180° oder 90° rotationssymmetrisch ausgebildet. Zusätzlich ist vorzugsweise eine Verriegelungsvorrichtung zum Verriegeln des Grundgeräts an der Aufnahmeplatte vorgesehen, um ein unbeabsichtigtes Lösen des Grundgeräts von der Aufnahmeplatte, beispielsweise aufgrund eines unbeabsichtigten Kippens des Trolleys, zu verhindern. Eine Aufnahmeplatte hat den Vorteil, dass eine definierte Lage des Grundgeräts zum Trolley leicht erzielbar ist. Zudem ist das Grundgerät leicht an der Aufnahmeplatte fixierbar, um ein unbeabsichtigtes Ablösen des Grundgeräts vom Trolley zu vermeiden.

Es ist erfindungsgemäß bevorzugt, dass das Grundgerät und die Aufnahmeplatte derart ausgebildet sind, dass das Grundgerät in zwei um 180° voneinander verschiedenen Positionen oder um mindestens drei, vorzugsweise vier, um 90° voneinander verschiedenen Positionen auf der Aufnahmeplatte anordenbar sowie fixierbar ist. Auf diese Weise sind zwei Konfigurationen des Beatmungsgeräts erzielbar, welche vorzugsweise zur Anordnung links und rechts neben einem Krankenhausbett optimiert sind. Ein für ein links neben einem Krankenhausbett konfiguriertes Beatmungsgerät ist somit leicht für die Verwendung rechts neben einem Krankenhausbett umkonfigurierbar, nämlich durch Drehung um 180° relativ zur Aufnahmeplatte. Weiter bevorzugt ist das Grundgerät insgesamt um drei oder vier um 90° voneinander verschiedenen Positionen auf der Aufnahmeplatte anordenbar sowie fixierbar Auf diese Weise sind drei bzw. vier Konfigurationen des Beatmungsgeräts erzielbar, welche vorzugsweise zur Anordnung links oder rechts neben, hinter oder vor einem Krankenhausbett optimiert sind. Ein für ein links neben einem Krankenhausbett konfiguriertes Beatmungsgerät ist somit leicht für die Verwendung vor oder hinter einem Krankenhausbett umkonfigurierbar, nämlich durch Drehung um 90° relativ zur Aufnahmeplatte. Auf diese Weise ist mit einfachen Mitteln sowie kostengünstig eine leichte Umkonfigurierbarkeit des Beatmungsgeräts verbessert.

Weiter bevorzugt ist die Benutzerschnittstelle linear verschiebbar an der Deckplatte, insbesondere linear verschiebbar am Schwenkgelenk, angeordnet. Die Benutzerschnittstelle ist vorzugsweise senkrecht und/oder parallel zur Schwenkachse linear verschiebbar. Vorzugsweise ist die Benutzerschnittstelle derart verschiebbar am Schwenkgelenk angeordnet, dass das Schwenkgelenk in einer ersten Position an einem Randbereich, insbesondere einem unteren Randbereich, der Benutzerschnittstelle angeordnet ist. Ferner ist die Benutzerschnittstelle vorzugsweise derart verschiebbar am Schwenkgelenk angeordnet, dass das Schwenkgelenk in einer zweiten Position an einem mittleren Bereich oder oberen Randbereich der Benutzerschnittstelle angeordnet ist. Dies kann beispielsweise über eine Linearführung realisiert werden. Vorzugsweise weist die Linearführung eine Rastvorrichtung zum Vermeiden einer unbeabsichtigten linearen Verstellung der Benutzerschnittstelle auf. Eine derartige Verschiebbarkeit hat den Vorteil, dass eine Benutzerfreundlichkeit des Beatmungsgeräts hierdurch verbesserbar ist. Somit ist beispielsweise eine Ausrichtung der Benutzerschnittstelle zu einem Benutzer des Beatmungsgeräts verbesserbar. Ferner ist hierdurch eine Verstaubarkeit des Beatmungsgeräts verbesserbar.

Gemäß einer bevorzugten Ausgestaltung der Erfindung kann bei einem Beatmungsgerät vorgesehen sein, dass mindestens ein Patientenanschluss schwenkbar an dem Grundgerät angeordnet ist. Schwenkbare Patientenanschlüsse haben den Vorteil, dass eine Ausrichtung von Beatmungsschläuchen zum Patienten verbesserbar ist, sodass eine ungewollte mechanische Belastung oder sogar ein Knicken der Beatmungsschläuche vermeidbar ist.

Vorzugsweise weist der Drehmechanismus eine zentrale Durchführung auf, durch welche Kabel zum elektrischen Koppeln der Benutzerschnittstelle mit dem Grundgerät hindurchführbar oder hindurchgeführt sind. Eine zentrale Durchführung hat den Vorteil, dass eine Bewegung der Kabel beim Drehen der Deckplatte relativ zum Grundgerät minimiert ist. Somit ist eine Beschädigung der Kabel auf kostengünstige Art und Weise sowie mit einfachen Mitteln vermeidbar.

### BEVORZUGTE AUSFÜHRUNGSBEISPIELE

Weitere, die Erfindung verbessernde Maßnahmen ergeben sich aus der nachfolgenden Beschreibung zu einigen Ausführungsbeispielen der Erfindung, welche in den Figuren dargestellt sind. Sämtliche aus den Ansprüchen, der Beschreibung oder der Zeichnung hervorgehende Merkmale und/oder Vorteile, einschließlich konstruktiver Einzelheiten und räumlicher Anordnungen können sowohl für sich, als auch in den verschiedenen Kombinationen erfindungswesentlich sein, wenn der resultierende Gegenstand nach Anspruch 1 ist. Es zeigen jeweils schematisch:
- Figur 1: eine perspektivische Ansicht einer bevorzugten Ausführungsform eines erfindungsgemäßen Beatmungsgeräts,
- Figur 2: eine perspektivische Ansicht eines kompakten Beatmungsgeräts nach dem Stand der Technik,
- Figur 3: eine perspektivische Ansicht eines flexibel konfigurierbaren Beatmungsgeräts nach dem Stand der Technik,
- Figur 4a-4c: perspektivische Ansichten unterschiedlicher Konfigurationen einer bevorzugten Ausführungsform eines erfindungsgemäßen Beatmungsgeräts,
- Figur 5a-5e: perspektivische Ansichten zweier Konfigurationen einer bevorzugten Ausführungsform eines erfindungsgemäßen Beatmungsgeräts mit Zwischenschritten, die den Vorgang des Umkonfigurierens zeigen,
- Figur 6a-6h: perspektivische Ansichten unterschiedlicher Anordnungen einer bevorzugten Ausführungsform eines erfindungsgemäßen Beatmungsgeräts an einem Patientenbett,
- Figur 7a-7c: perspektivische Ansichten einer bevorzugten Ausführungsform eines Drehmechanismus für ein erfindungsgemäßes Beatmungsgerät,
- Figur 8a-8c: perspektivische Ansichten einer weiteren bevorzugten Ausführungsform eines Drehmechanismus für ein erfindungsgemäßes Beatmungsgerät,
- Figur 9a-9d: Draufsicht und Schnittbilder einer bevorzugten Ausführungsform eines Schwenkgelenks für ein erfindungsgemäßes Beatmungsgerät,
- Figur 10a-10c: Draufsicht und Schnittbilder einer bevorzugten Ausführungsform eines erfindungsgemäßen Beatmungsgeräts mit Schwenkgelenk in Sperrposition,
- Figur 11a-11d: perspektivische Ansichten einer bevorzugten Ausführungsform eines erfindungsgemäßen Beatmungsgeräts mit verdeckten Geräteanschlüssen,
- Figur 12: eine perspektivische Ansicht einer bevorzugten Ausführungsform eines erfindungsgemäßen Beatmungsgeräts mit verdeckten Geräteanschlüssen auf einem Trolley,
- Figur 13a-13b: perspektivische Ansichten einer bevorzugten Ausführungsform eines erfindungsgemäßen Beatmungsgeräts mit verdeckten Geräteanschlüssen auf einer Tischplatte,
- Figur 14a-14c: perspektivische Ansichten einer weiteren möglichen Ausführungsform eines erfindungsgemäßen Beatmungsgeräts mit feststehender Benutzerschnittstelle, und
- Figur 15a-15b: perspektivische Ansichten einer weiteren möglichen Ausführungsform eines erfindungsgemäßen Beatmungsgeräts mit schwenkbarer Benutzerschnittstelle mit kleinen Abmessungen.

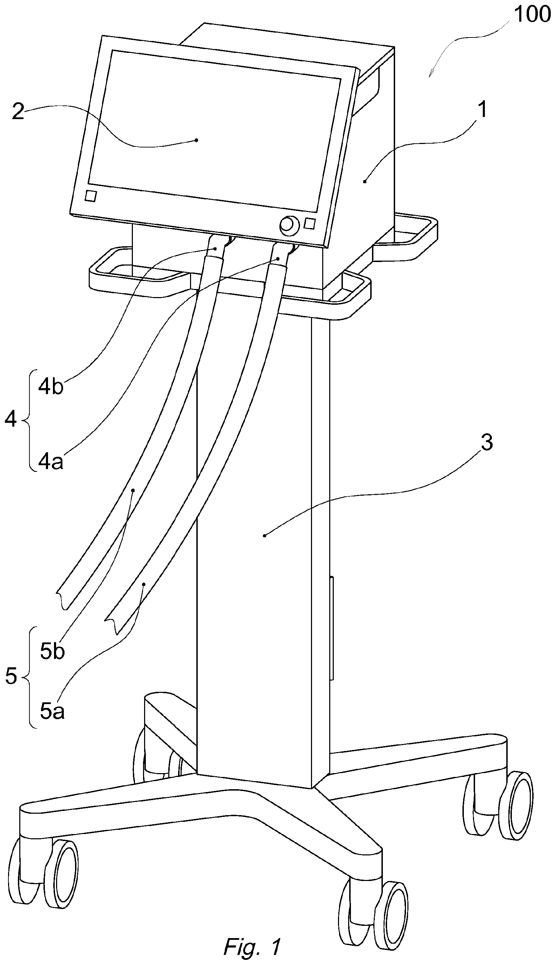

Elemente mit gleicher Funktion und Wirkungsweise sind in den Fig. 1 bis 15b jeweils mit denselben Bezugszeichen versehen.

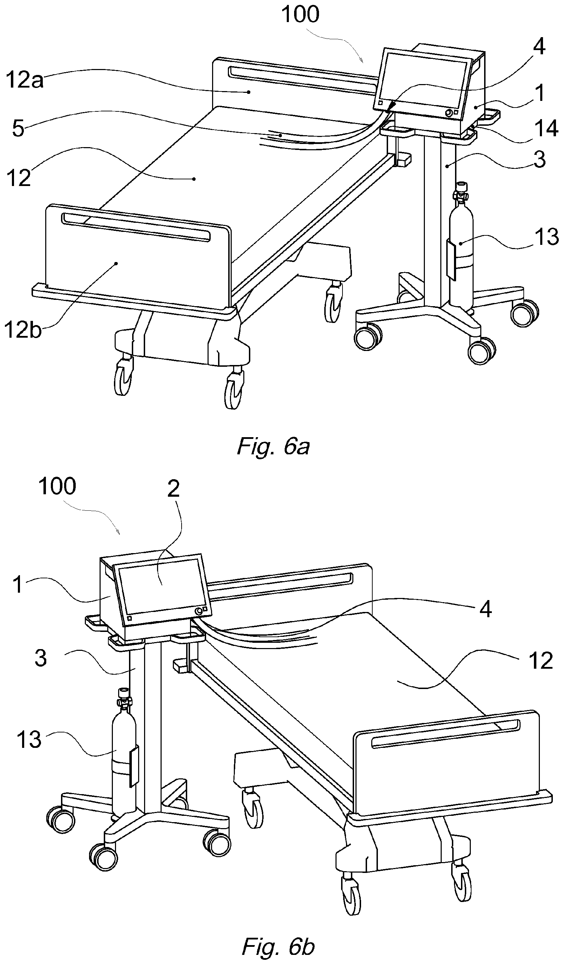

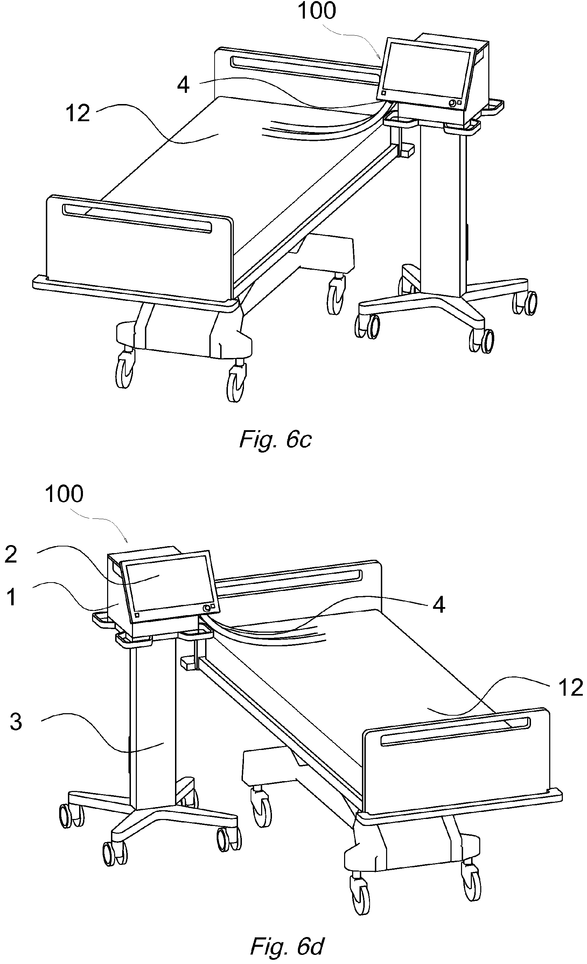

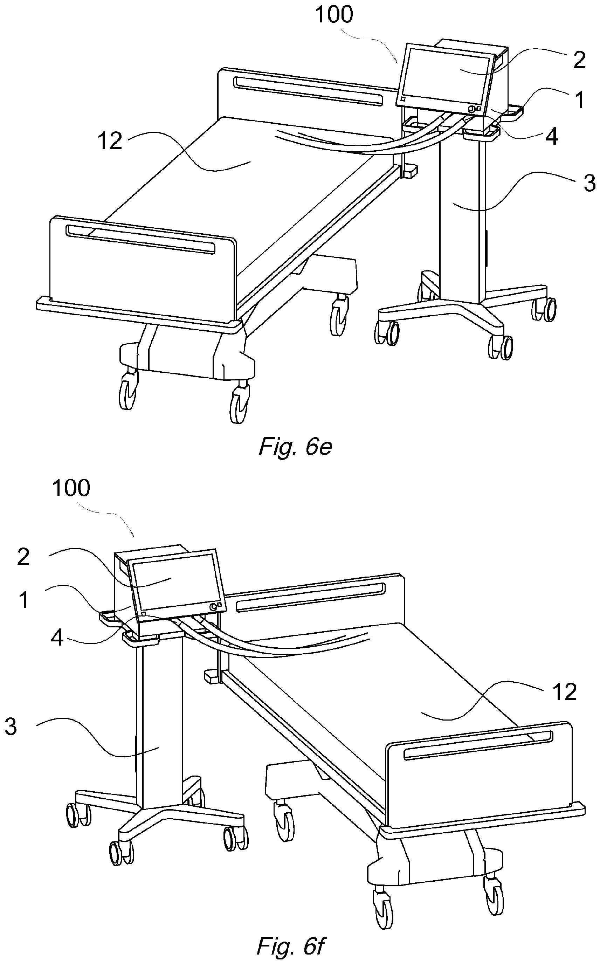

In Fig. 1 ist eine bevorzugte Ausführungsform eines erfindungsgemäßen Beatmungsgeräts 100 schematisch in einer perspektivischen Ansicht abgebildet. Gemäß dieser Ausführungsform weist das Beatmungsgerät 100 einen Trolley 3 auf, mit welchem ein Grundgerät 1 des Beatmungsgeräts 100 verbunden ist. Wenn das Gerät, wie in Fig. 1 gezeigt, auf einem Trolley 3 positioniert ist, findet man in Krankenhäusern hauptsächlich zwei Anordnungen vor: das Gerät steht rechts neben dem Patientenbett 12 (Fig. 6a, c, e) oder links neben dem Patientenbett 12 (Fig. 6b, d, f). Vorteilhaft ist bei beiden Anordnungen, wenn die Patientenanschlüsse 4 zum Patienten hin ausgerichtet sind, um die Längen der Beatmungsschläuche 5 möglichst kurz zu halten, während die Benutzerschnittstelle zum Fußende des Patientenbettes 12 ausgerichtet sein sollte, um vom Arzt gut eingesehen und bedient werden zu können.

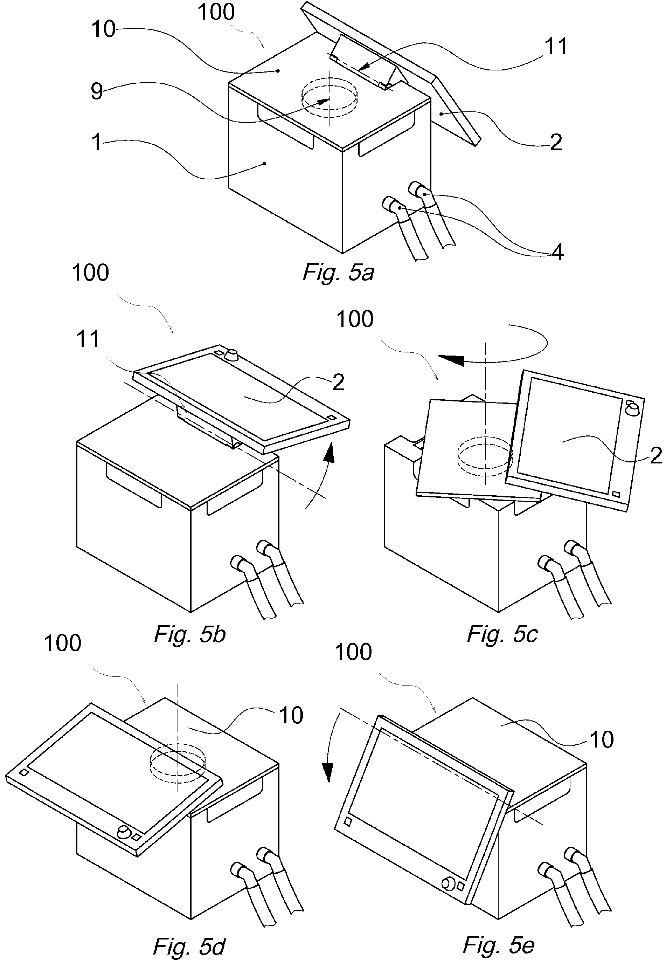

Das erfindungsgemäße Beatmungsgerät 100 weist gemäß der in Fig. 5a-e gezeigten speziellen Ausführungsform ein Grundgerät 1 mit seitlich angeordneten Patientenanschlüssen 4 und einer mittels eines vertikalen Drehmechanismus 9 darauf gelagerten Deckplatte 10 auf. An die Deckplatte 10 ist wiederum über ein Schwenkgelenk 11 eine Benutzerschnittstelle 2 angebunden. Um das Beatmungsgerät 100 von dem Zustand in Fig. 5a in den Zustand in Fig. 5e zu überführen, wird zunächst die Benutzerschnittstelle 2 - wie in Fig. 5b gezeigt - durch Betätigung des Schwenkgelenks 11 in eine zumindest annähernd horizontale Position nach oben geschwenkt, sodass sich die Benutzerschnittstelle 2 im Folgenden um 180° mittels der drehbar gelagerten Deckplatte 10 auf die gegenüberliegende Seite drehen lässt (Fig. 5c-d), ohne mit dem Grundgerät 1 zu kollidieren. Schließlich wird die Benutzerschnittstelle 2 auf dieser Seite des Grundgeräts 1 wieder mittels des Schwenkgelenks 11 in die gewünschte Winkelstellung heruntergeschwenkt (Fig.5e).

Ein Umkonfigurieren des Beatmungsgeräts 100, wie vorstehend beschrieben, sieht vorzugsweise zumindest einen 180° rotationssymmetrischen, ganz besonders bevorzugt rechteckigen Grundriss des Grundgeräts 1 und der Deckplatte 10 vor, damit die Deckplatte 10 in beiden Endstellungen einen zum Grundgerät 1 deckungsgleichen Grundriss einnehmen kann.

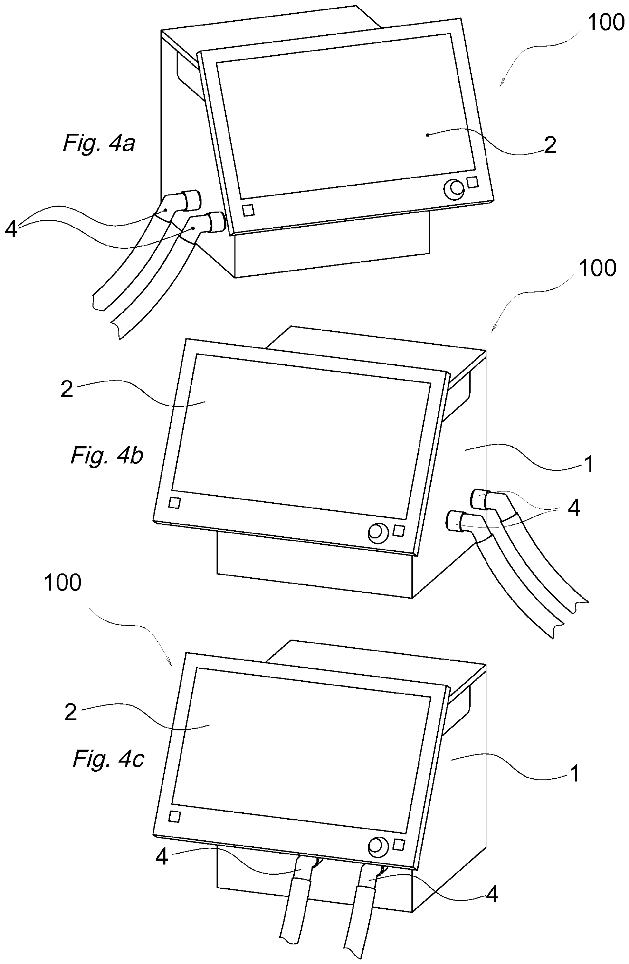

Eine erweiterte Funktionalität, die im Prinzip genauso funktioniert, kann gemäß einer weiteren Ausführungsform dadurch erreicht werden, dass sich die Deckplatte 10 in 90°-Schritten zum Grundgerät 1 drehen lässt (wie in Fig. 4a-c gezeigt). Dadurch ist auch eine Position der Benutzerschnittstelle 2 in gleicher Ausrichtung wie die Patientenanschlüsse 4 möglich, im Prinzip sogar auch eine vierte Position mit Benutzerschnittstelle 2 gegenüberliegend zu den Patientenanschlüssen 4. Für diese erweiterte Funktionalität muss der Grundriss des Grundgeräts 1 und der Deckplatte 10 um 90° rotationssymmetrisch sein, vorzugsweise quadratisch.

Der prinzipielle Aufbau des beschriebenen Mechanismus ist der gleiche wie bei einem Beatmungsgerät 100 nach Fig. 3. Die erfindungsgemäße Benutzerschnittstelle 2 ist ebenfalls mittels eines vertikalen Drehmechanismus 9 und daran anschließend durch ein horizontales Schwenkgelenk 11 an das Grundgerät 1 angebunden.

Der entscheidende Unterschied besteht in der Integration des vertikalen Drehmechanismus 9 in eine das Grundgerät 1 bündig abschließende Deckplatte 10 und in der nicht oberhalb des Grundgeräts 1, sondern vor dem Grundgerät 1 möglichst niedrig angebrachten Benutzerschnittstelle 2. Dadurch ergibt die Gesamtanordnung ein kompaktes Beatmungsgerät 100 mit geringer Bauhöhe trotz großer Benutzerschnittstelle 2. Ein Kabel 8, insbesondere ein Systemkabel, zur Verbindung der Benutzerschnittstelle 2 mit dem Grundgerät 1 wird auf vorteilhafte Weise innenliegend geführt, was hygienisch besser ist und den Eindruck eines kompakten Beatmungsgeräts 100 verstärkt.

Gemäß besonderen Weiterbildungen lässt sich die Benutzerschnittstelle 2 nur in 180°-Schritten (Gerät gemäß Fig. 5a-e) bzw. 90°-Schritten (Gerät gemäß Fig. 4a-c) zum Grundgerät 1 positionieren. Generell ist es allerdings auch denkbar, ein freies Drehen um die vertikale Achse zu ermöglichen, wie es auch mit dem Beatmungsgerät 100 gemäß Figur 3 möglich ist.

Gemäß der in Fig. 5a-e gezeigten Ausführungsform lässt sich das Beatmungsgerät 100 sowohl für eine Anordnung rechts vom Patientenbett 12 als auch links vom Patientenbett 12 konfigurieren, wobei es für die Ausrichtung der Patientenanschlüsse 4 zum Patienten unterschiedliche Möglichkeiten gibt: Entweder wird der Trolley 3 als Ganzes jeweils zum Patienten gewendet, wie in Fig. 6a und Fig. 6b gezeigt. Das ist von der Handhabung her am einfachsten, hat jedoch den Nachteil, dass am Trolley 3 angebrachtes Zubehör wie zum Beispiel ein Anfeuchter, ein Gelenkarm oder eine Gasflasche 13 nicht in beiden Positionen optimal angeordnet sein können und ggf. umgebaut werden müssen. Exemplarisch ist eine Gasflasche 13 mit Halter gezeigt.

Vorteilhaft ist es dagegen, wenn das Grundgerät 1 auf dem Trolley 3 ebenfalls umpositioniert bzw. umkonfiguriert werden kann, sodass der Trolley 3 in beiden Anordnungen dieselbe Position behält (Fig. 6c und Fig. 6d). Dazu ist gemäß einer speziellen Weiterbildung der Erfindung eine einfach lösbare Verbindung zwischen einer Aufnahmeplatte 14 des Trolleys 3 und dem Grundgerät 1 vorgesehen, die wiederum eine 180°-Rotationssymmetrie besitzt. Zum Umpositionieren wird das Grundgerät 1 vorzugsweise entriegelt, angehoben und um 180° versetzt wieder auf die Aufnahmeplatte 14 des Trolleys 3 aufgesetzt. Das anschließende Verriegeln kann selbsttätig erfolgen. Anwenderfreundlicher, aber auch konstruktiv aufwendiger, ist alternativ auch eine nicht dargestellte Drehvorrichtung denkbar, mittels welcher sich das Grundgerät 1 nach Betätigung einer Entriegelung auf dem Trolley 3 wenden lässt, bis es in der nächsten vorgesehenen Endstellung, also nach 180°, wieder selbsttätig einrastet.

Wenn das Beatmungsgerät 100 auch eine Konfiguration mit Patientenanschlüssen 4 und Bedienteil 2 in gleicher Ausrichtung zulässt wie in Fig. 4c gezeigt, dann besteht auch die Möglichkeit, das Grundgerät 1 stets in dieser Position zu lassen und die Anordnung zum Patientenbett 12 so zu gestalten wie in Fig. 6e und Fig. 6f gezeigt. So sind die Längen der Beatmungsschläuche 5 zwar nicht optimal und die Patientenanschlüsse 4 von der Zugänglichkeit her eingeschränkt, aber es entfällt die Notwendigkeit, das Beatmungsgerät 100 umzukonfigurieren, was in einigen Anwendungsfällen (häufige Patientenwechsel und häufiger Wechsel zwischen Position links- und rechtsseitig des Patientenbettes 12) vorteilhaft sein kann. Vorteilhafterweise sind die Patientenanschlüsse 4 bei einer solchen Anordnung als drehbare gewinkelte Tüllen ausgeprägt, die sich in einem gewissen Winkel zum Patienten hin ausrichten lassen, um die notwendigen Längen der Beatmungsschläuche 5 möglichst kurz halten zu können.

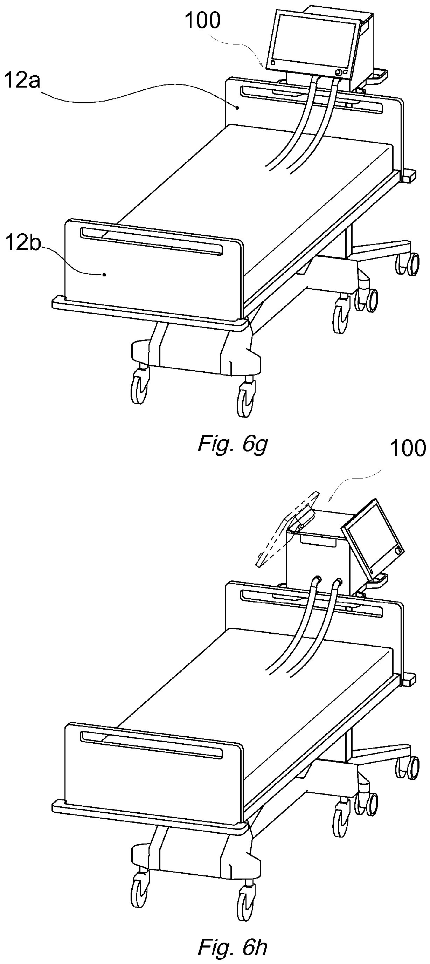

Auch für eine Position des Beatmungsgeräts 100 hinter einem Kopfende 12a des Patientenbetts 12 kann eine Gerätekonfiguration gemäß Fig. 4c vorteilhaft sein, da die Beatmungsschläuche 5 zum Patienten hin ausgerichtet sind und gleichzeitig die Benutzerschnittstelle 2 zu einem Fußende 12b des Patientenbetts 12 ausgerichtet ist, wo sie vom Arzt gut eingesehen werden kann, vgl. Fig. 6g. Tritt der Arzt dann nahe an den Kopf des Patienten, zum Beispiel um die Intubation zu überprüfen, kann es vorteilhaft sein, wenn er die Benutzerschnittstelle 2 temporär in eine Stellung links oder rechts von den Patientenanschlüssen 4 bringen kann, je nachdem auf welcher Seite des Patientenbetts 12 er steht, um sie besser ablesen und bedienen zu können, so wie in Fig. 6h gezeigt.

Um eine vollständige Konfigurierbarkeit des Verbundes aus Grundgerät 1 und Trolley 3 zu ermöglichen, die alle Anordnungen wie in Fig. 6a-h gezeigt ermöglicht und damit dem Anwender maximale Flexibilität bei Einrichten seines Arbeitsplatzes erlaubt, besitzt bevorzugt auch die Verbindung zwischen Aufnahmeplatte 14 des Trolleys 3 und Grundgerät 1 eine 90°-Rotationssymmetrie. Zum Umpositionieren wird das Grundgerät 1 vorzugsweise wiederum entriegelt, angehoben und je nach gewünschter Position um 90° oder 180° versetzt wieder auf den Trolley 3 aufgesetzt werden. Auch für dieses Ausführungsbeispiel kann alternativ eine Drehvorrichtung vorgesehen werden, mit der sich das Grundgerät 1 auf dem Trolley 3 wenden lässt, der in diesem Falle jedoch in 90°-Schritten einrastet.

Der Drehmechanismus 9 verfügt auf bevorzugte Weise, um die in Fig. 5a-e gezeigte Funktion sicher und benutzerfreundlich zu gestalten, zumindest eine der im Folgenden aufgeführten Eigenschaften auf, die sich auch kombinieren lassen:
- Fester Sitz der Deckplatte 10 in den definierten Gebrauchspositionen, sodass die Benutzerschnittstelle 2 möglichst spielarm mit dem Grundgerät 1 verbunden ist,
- Sperrung der vertikalen Drehbewegung, wenn die Benutzerschnittstelle 2 in einer der Gebrauchspositionen steht (Fig. 5a, Fig. 5e), um Fehlbedienung zu vermeiden,
- Leichtgängige Drehbewegung während des Drehens (Fig. 5c),
- Spürbares Einrasten bei Erreichen der Gebrauchspositionen,
- Drehanschläge an den Endpositionen (Fig. 5a, Fig. 5e), sodass der Drehmechanismus 9 auf einen definierten Winkelbereich, im Ausführungsbeispiel 180°, beschränkt bleibt. Ebenso kann eine entsprechende Beschränkung auf 360°, 540° oder 720° vorgesehen sein. Dadurch wird ein unkontrolliertes Verdrillen des Kabels 8 (Systemkabel oder mehrere Kabel), das das Grundgerät 1 mit der Benutzerschnittstelle 2 verbindet, verhindert,
- Keine Funktionselemente im Drehzentrum, da dieser Raum für eine Durchführung des Kabels 8 (Systemkabel oder mehrere Kabel) benötigt wird.

Im Folgenden werden zwei Ausführungsbeispiele gezeigt, in denen die zuvor genannten technischen Merkmale wenigstens teilweise umgesetzt werden. Die Ausführungsbeispiele beziehen sich dabei auf ein Gerät mit quadratischem Grundriss und drei möglichen Gebrauchspositionen der Benutzerschnittstelle wie in Fig. 4a-c, sind aber im Prinzip auch anwendbar auf ein Gerät mit nur zwei möglichen Gebrauchspositionen gemäß Fig. 5a-e.

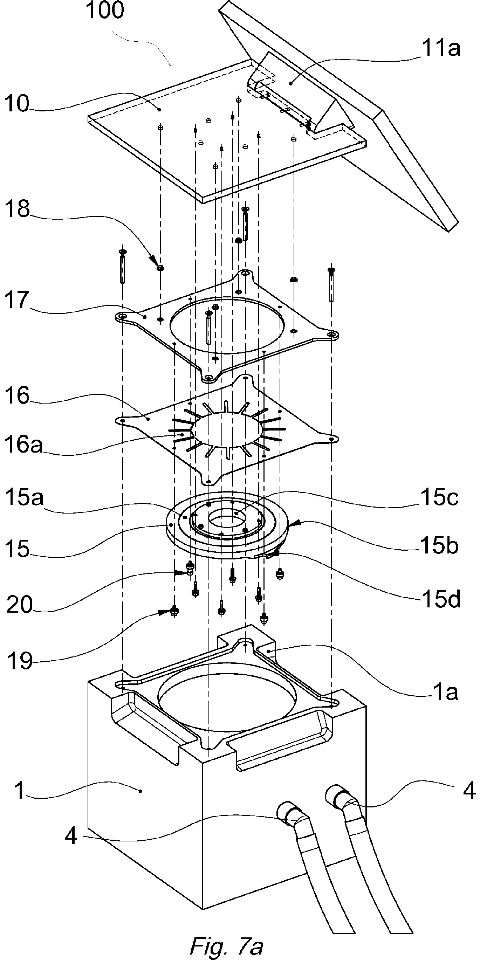

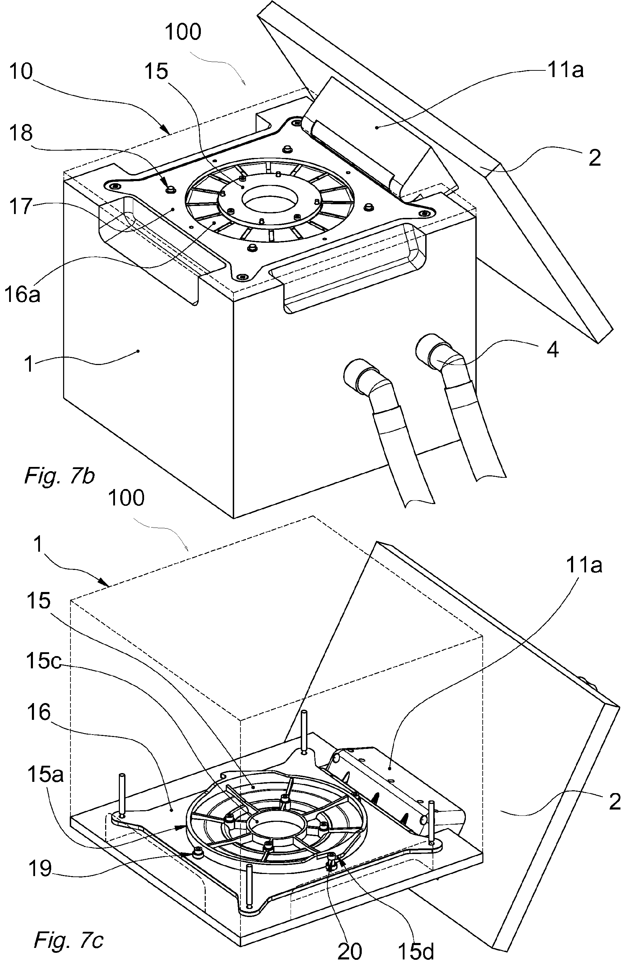

### Ausführungsbeispiel 1 (Fig. 7a-c)

Eine Gleitscheibe 15, insbesondere aus Kunststoff, ist fest mit der Deckplatte 10 verbunden, zum Beispiel verschraubt. Die Gleitscheibe 15 und damit auch die Deckplatte 10 werden im montierten Zustand über eine kreisförmig angeordnete Vielzahl von Blattfedern 16a, die zu einem Federblech 16 zusammengefasst sind, das wiederum mittels eines Halteblechs 17 fest mit dem Grundgerät 1 verbunden, zum Beispiel verschraubt ist, auf das Grundgerät 1 heruntergedrückt. Dazu liegen die Blattfedern 16a auf einer flach geneigten Kegelfläche 15a der Gleitscheibe 15 auf und gleiten bei Drehbewegung auch auf dieser ab. Zur Verringerung der Gleitreibung ist vorzugsweise eine Lebensdauerschmierung mit einem Fett vorgesehen.

Vier in die Deckplatte 10 eingelassene Rastnoppen 18, die zugleich als Gleitfüße ausgebildet sind, gleiten so lange über das Halteblech 17, bis eine der 90°-Positionen erreicht ist, dann fallen sie, getrieben von den Blattfedern 16a, in komplementäre Senklöcher in dem Halteblech 17 ein und bilden so die gewünschte Einrastfunktion. Auch diese Gleitfunktion kann durch eine Lebensdauerschmierung mit einem Fett verbessert werden. Die Blattfedern 16a haben auch in dieser Stellung des Mechanismus noch eine Vorspannung und wirken über einen relativ großen Durchmesser, sodass eine gute Abstützung der Deckplatte 10 zum Grundgerät 1 gegeben ist. Durch diese Ausführung der Einrastfunktion im Zusammenspiel mit der Wirkung der Blattfedern 16a hebt sich die Deckplatte 10 beim Drehen um einen gewissen Betrag, zum Beispiel 1 mm, vom Grundgerät 1 ab, sodass nur die dafür vorgesehenen Lagerungselemente im Eingriff sind, während ein Anstreifen zwischen Grundgerät 1 und Deckplatte 10 verhindert wird.

Die radiale Lagerung geschieht über vier als Kugellager ausgebildete Stützrollen 19, die auf dem Halteblech 17 befestigt sind und auf einer äußeren Zylinderfläche 15b der Gleitscheibe 15 abrollen. Eine einfache Lagerung über einen zentralen Zapfen wurde bewusst nicht gewählt, um im Drehzentrum Raum für eine Durchführung 27 zur Durchführung von Kabeln 8 zu lassen. Hierfür ist in der Gleitscheibe 15 eine zentrale Öffnung 15c vorgesehen.

Ein mit einer der Stützrollen 19 verbundener Anschlagstift 20, der als Schraubenkopf ausgebildet ist, bildet zusammen mit zwei an der Gleitscheibe 15 angeformten Anschlagnasen 15d die gewünschten Endanschläge für den Drehmechanismus 9.

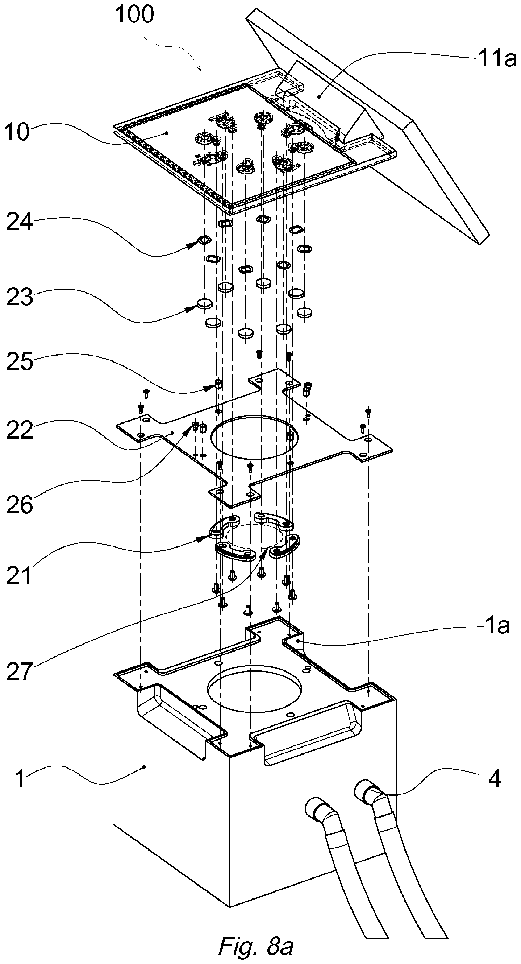

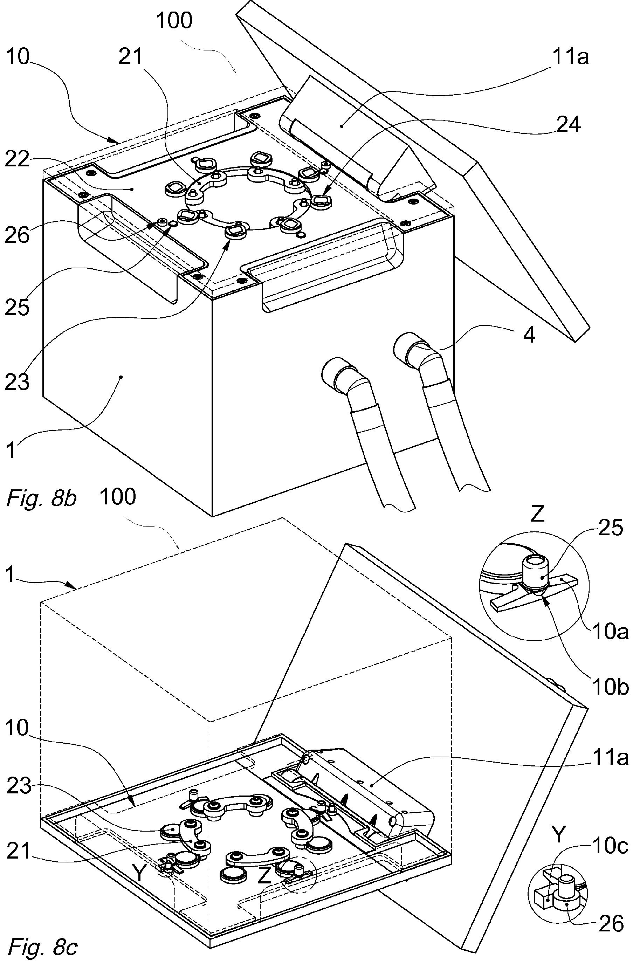

### Ausführungsbeispiel 2 (Fig. 8a-c)

Ein sowohl axial als auch radial wirkender Gleitlagerring 21 aus einem gleitfähigen Kunststoff, der im Beispiel in vier Segmente unterteilt ist, um die Außendurchmesser-Toleranz genauer einstellen zu können als es mit einem einteiligen Kunststoffring möglich wäre, ist fest mit der Deckplatte 10 verbunden, zum Beispiel verschraubt. Die komplementären axialen und radialen Laufflächen werden von einem Lagerblech 22 gebildet, das mit dem Grundgerät 1 fest verbunden, zum Beispiel verschraubt ist.

Um eine in axialer Richtung spielfreie Lagerung zu ermöglichen, drückt eine kreisförmig angeordnete Vielzahl von Gleitsteinen 23 mittels darüber liegenden Federscheiben 24, zum Beispiel Wellscheiben, die Deckplatte 10 von dem Lagerblech 22 ab.

Ein Einrasten in 90°-Schritten erfolgt durch zwei oder vier handelsübliche erste Kugelrasten 25, die kurz vor Erreichen der jeweiligen Gebrauchsposition über in die Deckplatte 10 eingeformte Rampen 10a eingedrückt und dann bei Erreichen der Gebrauchsposition in zu den Kugeln der ersten Kugelrasten 25 komplementäre Vertiefungen 10b einrasten.

Die Endanschläge werden im Ausführungsbeispiel durch zwei mit dem Lagerblech 22 verbundene Schraubenköpfe 26 gebildet, die bei Erreichen der jeweiligen Endposition an einen in die Deckplatte 10 eingeformten Vorsprung 10c anschlagen. Alle Laufflächen des Mechanismus können für eine bessere Leichtgängigkeit mit einem auf die Produktlebensdauer ausgelegten Fett geschmiert werden. Auch dieser Mechanismus weist eine Durchführung 27 zur Durchführung eines oder mehrerer Kabel 8, insbesondere Systemkabel, durch das Drehzentrum auf, da hier ein gewisser Durchmesser ausgespart ist.

### Sperrung der vertikalen Drehbewegung (Fig. 7a, Fig. 8a)

Die Sperrung der Drehbewegung bei heruntergeschwenkter Benutzerschnittstelle 2 ist bei beiden vorstehenden Ausführungsbeispielen dadurch realisiert, dass das Schwenkgelenk-Gehäuse 11a in eine am Grundgerät 1 vorgesehene Griffmulde 1a einschwenkt, sodass eine vertikale Drehbewegung durch Formschluss unterbunden ist. Alternativ oder unterstützend hierzu kann ein innenliegender Mechanismus realisiert werden, der bei heruntergeschwenkter Benutzerschnittstelle 2 den Drehmechanismus 9 blockiert, zum Beispiel über eine nach Art eines Kurbeltriebs in den Mechanismus einfahrende Schubstange oder eine beim Herunterschwenken ausfahrende Sperrklinke (keine Abbildungen verfügbar).

Das Schwenkgelenk 11, das für ein Bedienelement, einen Monitor und/oder ein Display, vorzugsweise für eine Benutzerschnittstelle 2 mit Touch-Funktion und eventuellen weiteren Bedienelementen auf bevorzugte Weise geeignet ist und außerdem eine annähernd horizontale Position der Benutzerschnittstelle 2, wie in Fig. 5b gezeigt, ermöglicht, weist vorteilhafterweise wenigstens eines der folgenden konstruktiven Merkmale auf, die wiederum auch bedarfsgerecht kombiniert werden können:
- Verzicht auf ein Entriegelungselement, das betätigt werden müsste, um den Schwenkwinkel zu verändern (vorteilhaft in Bezug auf Bedienkomfort und Hygiene)
- Stattdessen ein Friktionselement, dessen Reibmoment groß genug ist, um typische Kräfte, die der Benutzer bei Betätigung der Touch-Funktion oder weiterer Bedienelemente auf das Bedienteil ausübt, so weit abzustützen, dass kein versehentliches Verstellen des Schwenkwinkels erfolgt.
- Ein Federelement zur Kompensation des durch die Gewichtskraft der Benutzerschnittstelle auf das Schwenkgelenk 11 wirkenden Momentes, sodass die Summe aus Reibmoment, Gewichtsmoment und Federmoment beim Hoch- und Herunterschwenken zumindest annähernd gleich ist.
- Optional ein Rastelement, das statt einer kontinuierlichen eine schrittweise Verstellbarkeit des Schwenkwinkels bewirkt, und/oder durch das sich der als Gebrauchsposition vorgesehene Schwenkwinkelbereich haptisch deutlich von der annähernd horizontalen Position (Fig. 5b), die nur zum Umkonfigurieren des Beatmungsgeräts 100 gemäß Fig. 5a-e dienen soll, unterscheiden lässt.

Die vorstehenden Eigenschaften werden durch das im Folgenden gezeigte Ausführungsbeispiel abgedeckt. Das Ausführungsbeispiel bezieht sich dabei auf ein Grundgerät 1 mit quadratischem Grundriss und drei möglichen Gebrauchspositionen der Benutzerschnittstelle 2 wie in Fig. 4a-c, ist aber im Prinzip auch anwendbar auf ein Grundgerät 1 mit nur zwei möglichen Gebrauchspositionen gemäß Fig. 5a-e.

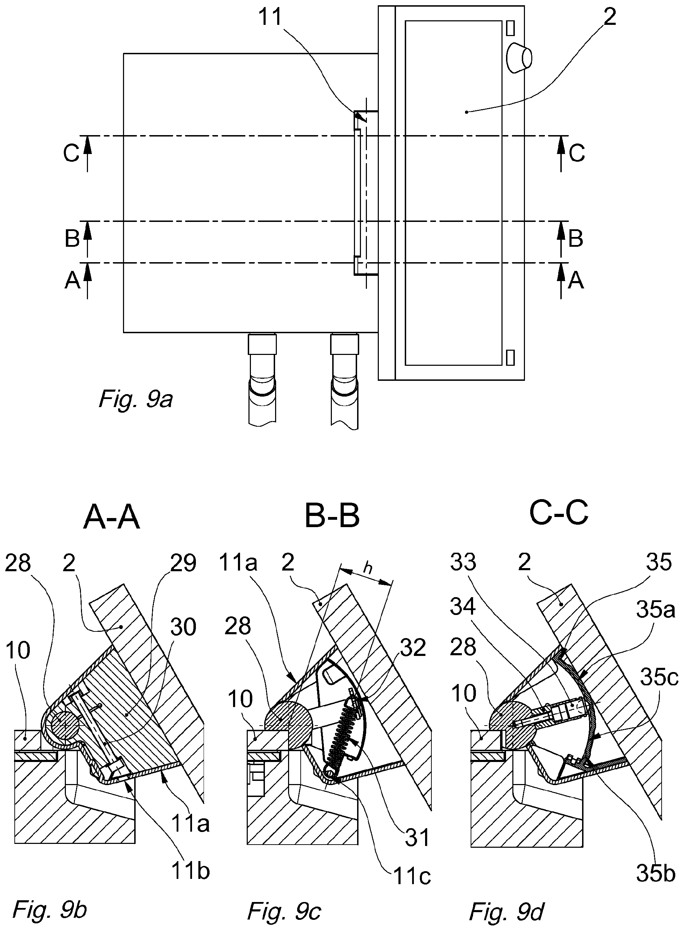

### Ausführungsbeispiel (Fig. 9a-d):

Ein beispielhaftes Schwenkgelenk 11 ist in drei Schnittebenen gezeigt, die in Fig. 9a definiert sind. Die Schnittebene A-A (Fig. 9b) zeigt ein im Ausführungsbeispiel zweimal vorhandenes Friktionselement, bestehend aus einem fest mit der Deckplatte 10 verbundenen, vorzugsweise aus Metall bestehenden Achsstück 28 und einem fest mit der Benutzerschnittstelle 2 verbundenen Lagerblock 29, vorzugsweise aus Kunststoff, dessen Lagerbohrung eine mittels einer Klemmschraube 30 einstellbare Presspassung zum Achsstück 28 in der Art bildet, dass sich das Reibmoment durch Festziehen bzw. Lösen der Klemmschraube 30 einstellen lässt. Eine Gehäuseöffnung 11b im Schwenkgelenk-Gehäuse 11a erlaubt dabei das Einstellen auch im montierten Zustand.

In der Schnittebene B-B (Fig. 9c) ist ein im Ausführungsbeispiel viermal vorhandenes Federelement dargestellt, bestehend aus einer handelsüblichen Zugfeder 31, die mit ihrem oberen Ende an einer mit dem Achsstück 28 und damit mit der Deckplatte 10 verbundenen Haltestruktur 32 und mit ihrem unteren Ende an einem an das Schwenkgelenk-Gehäuse 11a angeformten Gegenlager 11c eingehängt ist. Das Schwenkgelenk-Gehäuse 11a ist wiederum mit der Benutzerschnittstelle 2 fest verbunden. Durch die Wirkung der vorgespannten Zugfedern 31 wird zumindest im als Gebrauchsposition vorgesehenen Winkelbereich der Benutzerschnittstelle 2 (zum Beispiel 0° bis 45° gegen vertikal) eine annähernd gleichstarke Kompensation der Gewichtskraft der Benutzerschnittstelle 2 erreicht, da durch die gewählte Anordnung die wirksame Hebellänge h um die Schwenkachse des Schwenkgelenks 11 sich beim Hochschwenken der Benutzerschnittstelle 2 vergrößert, während die Vorspannkraft der Zugfeder 31 zugleich abnimmt.

In der Schnittebene C-C (Fig. 9d) ist ein im Ausführungsbeispiel sechsmal vorhandenes Rastelement gezeigt, bestehend aus einer handelsüblichen zweiten Kugelraste 33, die in eine mit dem Achsstück 28 und damit mit der Deckplatte 10 fest verbundene, zum Beispiel verschraubte Hülse 34 eingelassen ist, und einer Rastkulisse 35 mit einer Vielzahl von zu der Kugel der zweiten Kugelraste 33 komplementären ersten Einbuchtungen 35a, die die Raststellungen für die vorgesehenen Gebrauchsstellungen definieren (im Beispiel in 5°-Schritten von 0° bis 45°). Eine weitere Einbuchtung 35b hält die Benutzerschnittstelle 2 in der annähernd horizontalen Position gemäß Fig. 5b fest, um nach Einstellen dieser Position ein Umkonfigurieren des Beatmungsgeräts 100 gemäß Fig. 5a-e zu ermöglichen. Da die Kugel der zweiten Kugelraste 33 zwischen den ersten Einbuchtungen 35a weniger weit eingedrückt wird als in dem Bereich 35c ohne erste Einbuchtungen 35a, ist die Verstellung zwischen den vorgesehenen Gebrauchsstellungen spürbar leichtgängiger als der Übergang zur annähernd horizontalen Position. Dadurch kann der Anwender haptisch gut zwischen den normalen Gebrauchsstellungen und der besonderen, annähernd horizontalen Stellung, die für die Umkonfiguration des Beatmungsgerätes 100 eingenommen werden muss, unterscheiden.

### Mögliche alternative Ausführungsformen für ein Friktionselement:

Anstatt einer mit radialer Pressung funktionierenden Reibpaarung wie im obenstehenden Ausführungsbeispiel (Fig. 9b) sind auch eine oder mehrere axial wirkende Reibbremsen nach dem Wirkprinzip einer Lamellenkupplung oder eine oder mehrere kegelförmige Reibpaarungen, die die Reibwirkung durch die Keilwirkung des Kegelwinkels erhöhen, denkbar, wobei eine definierte Andrückkraft und daraus resultierendes Reibmoment jeweils zum Beispiel über eine oder mehrere konzentrisch zu dem Achsstück 28 angeordnete entsprechend vorgespannte Druckfedern oder Tellerfedern erzeugt werden könnte.

### Mögliche alternative Ausführungsformen für ein Federelement:

Zur Kompensation des Moments, das aus der Gewichtskraft der Benutzerschnittstelle 2 resultiert, kann anstatt einer Anordnung aus einer oder mehreren Zugfedern 31 wie in Fig. 9c gezeigt auch zum Beispiel eine oder mehrere vorgespannte Schenkelfedern oder Wickelfedern verwendet werden. Letztere hat die besondere Eigenschaft, über einen größeren Winkelbereich ein konstantes Drehmoment zur Verfügung zu stellen, was für die gewünschte Kompensation von Vorteil ist gegenüber einer Feder, deren Drehmoment beim Hochschwenken des Bedienteils abnimmt.

### Mögliche alternative Ausführungsformen für ein Rastelement:

Eine oder mehrere zweite Kugelrasten 33 können anstatt in radialer Richtung wie im Ausführungsbeispiel (Fig. 9d) auch in axialer Richtung angeordnet sein. Zweite Kugelrasten 33 und komplementäre erste Einbuchtungen 35a sind dann in einer Achse parallel zur Mittelachse des Achsstücks 28 angeordnet, wobei diese Achsen möglichst weit voneinander entfernt sind, sodass das durch die Rastfunktion bewirkte Haltemoment möglichst groß wird. Eine Anordnung wie in Fig. 9d gezeigt kann vorzugsweise anstatt einer Rastkugeln aufweisenden zweiten Kugelraste 33 auch mit einem oder mehreren zylindrischen, durch Federkraft beaufschlagten Raststäben ausgebildet sein, die senkrecht zur Schnittebene von Fig. 9d eine größere Länge haben. Die komplementären ersten Einbuchtungen 35a und die weitere Einbuchtung 35b, in die der oder die Raststäbe einfallen, haben dann eine entsprechende zylindrische Rillenform. Vorteil einer solchen Ausprägung wären die gegenüber einer kugelförmigen Einbuchtung größeren Wirkflächen und damit geringere Verschleißneigung der Einbuchtungen.

### Begrenzung des Schwenkwinkels der Benutzerschnittstelle nach unten

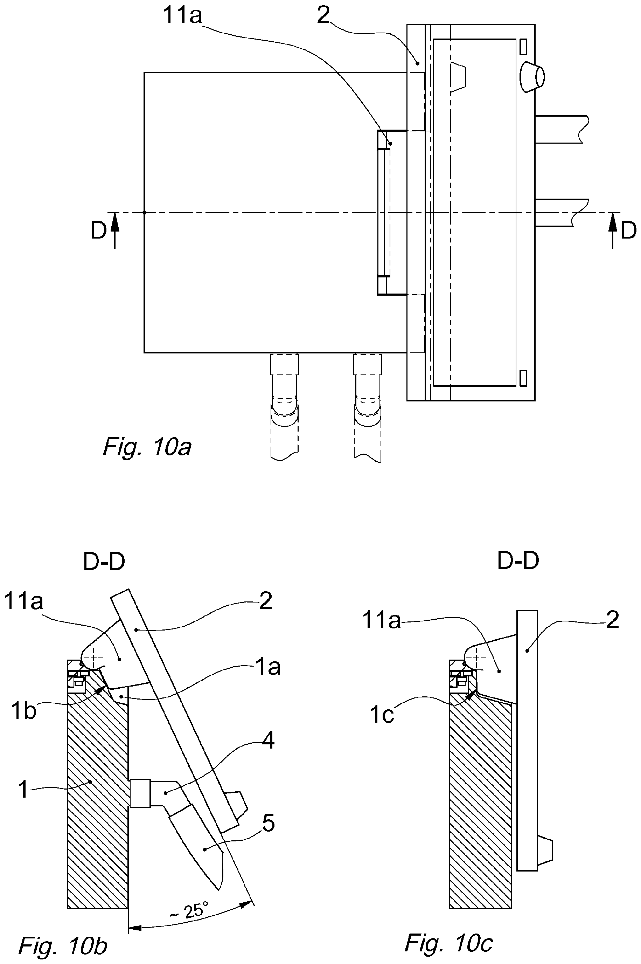

Eine Besonderheit des Ausführungsbeispiels für ein Beatmungsgerät 100 mit einem Grundgerät 1 mit einem quadratischen Grundriss und drei möglichen Gebrauchspositionen der Benutzerschnittstelle 2 wie in Fig. 4a-c dargestellt ist, dass sich die Benutzerschnittstelle 2 in einer Konfiguration des Beatmungsgeräts 100 mit Patientenanschlüssen 4 in derselben Ausrichtung wie die Benutzerschnittstelle 2 (Fig. 4c) nur bis zu einem gewissen Winkel, zum Beispiel 25° gegen vertikal, herunterschwenken lässt, weil es sonst mit den Patientenanschlüssen 4 und/oder den Beatmungsschläuchen 5 kollidieren würde, so wie in Fig. 10b gezeigt. Dagegen lässt sich die Benutzerschnittstelle 2 in einer Konfiguration des Beatmungsgeräts 100 mit Patientenanschlüssen 4 seitlich zur Benutzerschnittstelle 2 (Fig. 4a oder Fig. 4b) bis in eine zumindest annähernd vertikale Position schwenken, so wie in Fig. 10c dargestellt.

Um den gesamten Schwenkwinkelbereich der Benutzerschnittstelle 2 in einer Gerätekonfiguration gemäß Fig. 4a oder Fig. 4b nutzen zu können, ihn aber gleichzeitig in einer Konfiguration gemäß Fig. 4c so zu begrenzen, dass eine Kollision mit den Patientenanschlüssen 4 vermieden wird, sind die im Grundgerät 1 eingeformten Griffmulden 1a im Ausführungsbeispiel unterschiedlich ausgeformt: die Griffmulde 1a, die zu den Patientenanschlüssen 4 hin ausgerichtet ist, weist eine gewinkelte Anschlagfläche 1b für das Schwenkgelenk-Gehäuse 11a auf, durch die das Herunterschwenken der Benutzerschnittstelle 2 so begrenzt wird, dass eine Kollision mit den Patientenanschlüssen 4 vermieden wird. Die bezüglich der Patientenanschlüsse 4 seitlichen Griffmulden 1a weisen dagegen zumindest annähernd senkrechte Anschlagflächen 1c auf, die ermöglichen, dass die Benutzerschnittstelle 2 in eine zumindest annähend senkrechte Lage heruntergeschwenkt werden kann.

Die vorgenannten Ausführungsbeispiele für Drehmechanismen und Schwenkgelenke stellen keine Beschränkung des allgemeinen Erfindungsgedankens dar. Insbesondere ist es diesbezüglich denkbar, die unterschiedlichen technischen Merkmale miteinander zu kombinieren, sodass nicht nur besonders geeignete Schwenkgelenke und Drehmechanismen, sondern auch entsprechende Kombinationen von Drehmechanismen und Schwenkgelenken zur Verfügung gestellt werden bzw. unter die Erfindung subsummiert werden können.

Eine Rückseite eines Beatmungsgeräts 100 hat typischerweise geringere dekorative und auch hygienische Anforderungen, sodass sie auf bevorzugte Weise für die Platzierung wenigstens einer, bevorzugt einer Mehrzahl oder aller Schnittstelle/n für Kabel 8 und Schläuche, die fest installiert werden, wie zum Beispiel Gaseingangsschläuche, Netzkabel, Netzwerkkabel, und für weitere Funktionselemente wie Hauptschalter, Lüftungseinlässe oder Akkumulator-Einschübe verwendet wird.

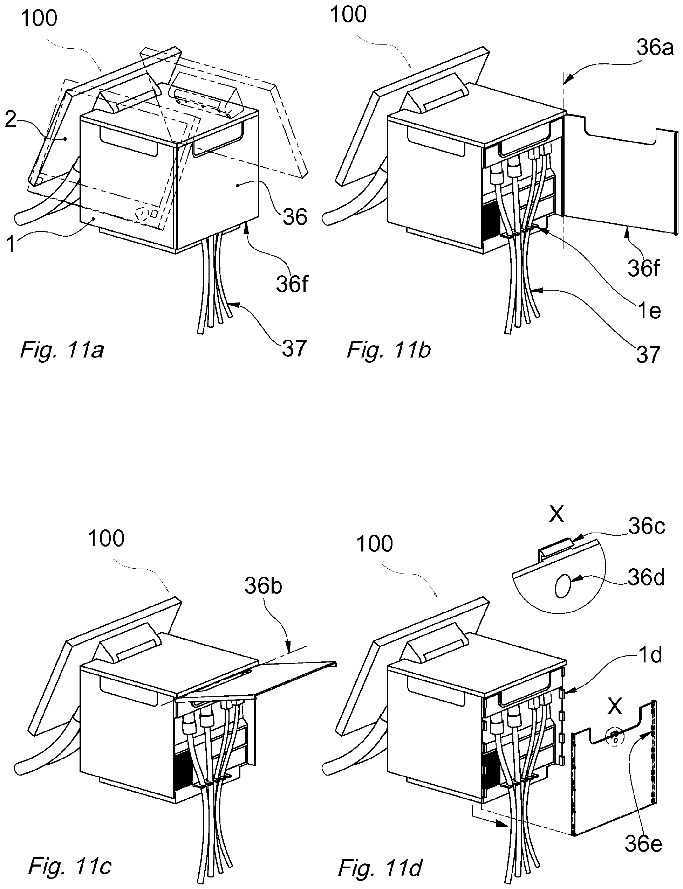

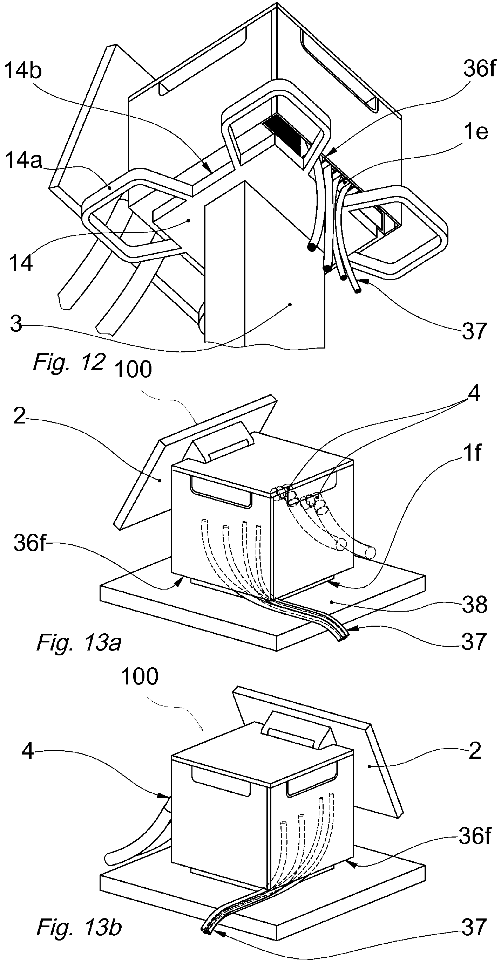

In der besonderen Ausgestaltung zeichnet sich ein erfindungsgemäßes Beatmungsgerät 100, das sich gemäß Fig. 4a-c oder Fig. 5a-e umkonfigurieren lässt, dadurch aus, dass das Grundgerät 1 keine solche Rückseite in dem Sinne hat, dass sie immer die dem Anwender abgewandte Seite ist. Daher kann ein solches Beatmungsgerät 100 vorteilhafterweise eine die Form des Grundgeräts 1 abschließende Designabdeckung 36 aufweisen, die je nach Gerätekonfiguration bezüglich der Benutzerschnittstelle 2 die linke oder die rechte Geräteseite oder die Rückseite bildet. Eine solche Designabdeckung 36 ist beispielhaft in Fig. 11a abgebildet. Die Designabdeckung 36 muss vom Anwender leicht zu öffnen oder abzunehmen sein, um die oben beschriebenen Kabel- und Schlauchschnittstellen und weiteren Funktionselemente zugänglich zu machen. Dafür kann die Designabdeckung 36 zum Beispiel als Tür mit vertikaler Achse, wie in Fig. 11b gezeigt, oder mit in der Nähe der Oberkante angeordneter, horizontaler Achse 36b ausgeprägt sein oder z. B. durch Entriegeln eines Riegelelements 36c und Herunterschieben und damit Herausfahren aus einem zum Beispiel durch abschnittsweise ausgebildete Nut- und Federelemente 1d gebildeten Formschluss lösbar sein. Das Herausführen von Kabeln und Schläuchen 37 sowie das Ansaugen von Luft für Gerätekühlung oder auch für einen Beatmungs-Blower erfolgt vorteilhafterweise durch eine Öffnung an der Unterkante 36g der Designabdeckung 36, sodass keine weiteren Öffnungen sichtbar sind. Diese Öffnung kann kammartige Elemente 1e aufweisen, um die Führung der Kabel 8 und Schläuche zu definieren. Im Systemverbund mit einem Trolley 3 lassen sich so die Kabel 8 und Schläuche im Wesentlichen unabhängig von einer Platzierung des Grundgeräts 1 auf dem Trolley 3, wie in Fig. 12a gezeigt, nach unten wegführen. An der Aufnahmeplatte 14 sind in diesem Ausführungsbeispiel an allen Seiten Griffe 14a angeordnet, welche auch als Schienen mit genormtem Profil zum Anbringen von Zubehör, wie zum Beispiel einem Gelenkarm, ausgebildet sein können. Zudem weist die Aufnahmeplatte 14 vorzugsweise an mindestens drei Seiten mittige Aussparungen 14b auf, sodass sich die Kabel und Schläuche 37 kollisionsfrei nach unten führen lassen, unabhängig davon, ob die Patientenanschlüsse 4 am Grundgerät 1 relativ zum Trolley 3 nach vorne, nach links oder nach rechts positioniert sind (vgl. Fig. 6c-f).

Eine Besonderheit ist dagegen die Verwendung des erfindungsgemäßen Beatmungsgeräts 100 als Tischgerät: die Unterkante 36f der Designabdeckung 36 ist in diesem Falle mittels eines am Grundgerät 1 angeformten Sockels 1f so weit von einer Tischplatte 38 beabstandet, dass sich alle Kabel und Schläuche 37 seitlich - bezüglich der Abdeckung - nach links oder rechts herausführen lassen, ohne übermäßig geknickt zu werden. Dadurch lassen sich die beiden Gerätekonfigurationen mit - bezüglich der Benutzerschnittstelle 2 - auf der linken Seite (vgl. Fig. 12b) bzw. der rechten Seite (vgl. Fig. 12c) angeordneten Patientenanschlüssen 4 auch als Tischgerät nutzen, indem sich die Kabel und Schläuche 37 in beiden Konfigurationen bezüglich der Benutzerschnittstelle 2 nach hinten wegführen lassen.

Von dem erfindungsgemäßen Beatmungsgerät 100 gemäß Fig. 4a-c oder Fig. 5a-e lassen sich einfachere Gerätevarianten ableiten, indem das beschriebene Grundgerät 1 als gemeinsame Basis verwendet wird, während die beschriebene dreh- und schwenkbare Funktionsbaugruppe, umfassend die Deckplatte 10, das Schwenkgelenk 11 und die Benutzerschnittstelle 2, durch ein vereinfachtes, zum Beispiel nicht bewegliches oder nur schwenkbares Bedienmodul ersetzt wird.

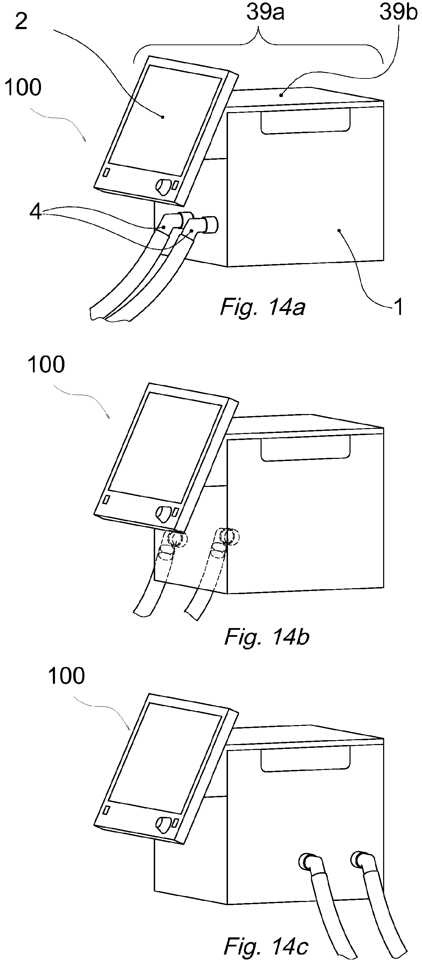

Fig. 14a zeigt ein vereinfachtes Beatmungsgerät 100 mit einer kleineren, oberhalb der Patientenanschlüsse 4 angebrachten Benutzerschnittstelle 2, die zusammen mit einer fest mit dieser verbundenen Deckplatte 39b ein Bedienmodul 39a bildet. Die Deckplatte 39b ist in diesem Ausführungsbeispiel fest mit dem Grundgerät 1 verbunden. Diese Gerätevariante entspricht im Prinzip einem kompakten Beatmungsgerät 100 gemäß Fig. 2. Wenn das Grundgerät 1 einen um 90° rotationssymmetrischen Grundriss aufweist wie in Fig. 4a-c dargestellt, kann das Bedienmodul 39a stattdessen in einer um 90° nach rechts (vgl. Fig. 14b) oder nach links (vgl. Fig. 14c) versetzten Position fest mit dem Grundgerät 1 verbunden werden, sodass der Anwender je nach bevorzugtem Anwendungsfall (vgl. Fig. 6c-f) die Wahl zwischen einem Beatmungsgerät 100 mit Patientenanschlüssen 4 nach vorne, nach links oder nach rechts hat. Die Konfiguration kann werksseitig oder zum Beispiel durch einen Servicetechniker beim Anwender erfolgen.

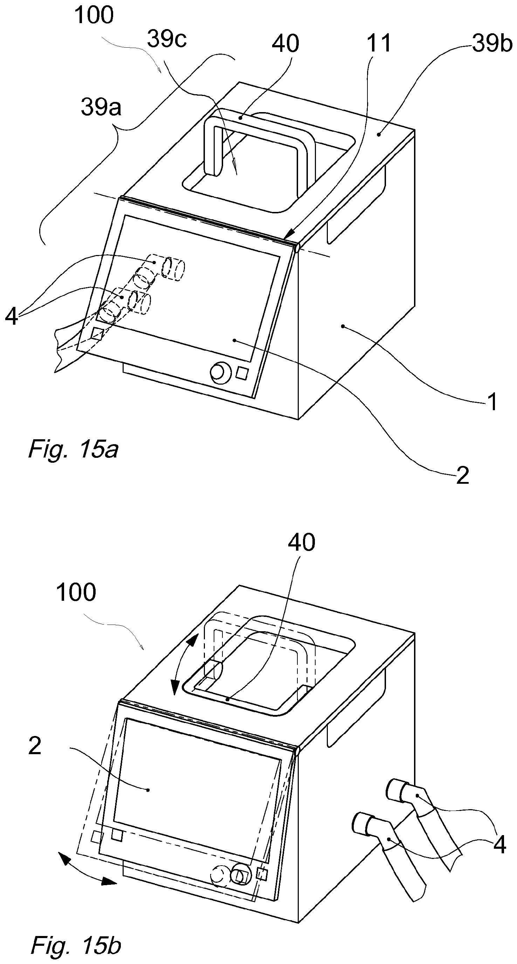

Fig. 15a zeigt ein besonders kompaktes Beatmungsgerät 100, dessen Bedienmodul 39a eine noch kleinere Benutzerschnittstelle 2 aufweist, die seitlich zu den Patientenanschlüssen 4 derart angeordnet ist, dass diese die Oberseite der Deckplatte 39b nicht überschreitet. Vorzugsweise weist die Benutzerschnittstelle 2 dieselbe Breite wie das Grundgerät 1 auf, wodurch die Kompaktheit des Beatmungsgeräts 100 unterstrichen wird. Zwischen der Benutzerschnittstelle 2 und der Deckplatte 39b ist vorzugsweise ein Schwenkgelenk 11 angeordnet, sodass der Winkel der Benutzerschnittstelle 2 in eine für den Anwender vorteilhafte Position einstellbar ist. Auf der Deckplatte 39b kann ein Tragegriff 40 zum Tragen des Beatmungsgeräts 100 angeordnet sein. Der Tragegriff 40 ist vorzugsweise um eine horizontale Achse verschwenkbar bzw. ausklappbar und vorzugsweise derart an der Deckplatte 39b gehalten, dass dieser in einem eingeklappten Zustand in einer Mulde 39c der Deckplatte 39b versenkbar ist. Ein verschwenkbarer Tragegriff 40 hat den Vorteil, dass dieser im eingeklappten Zustand eine Bauhöhe des Beatmungsgeräts 100 nicht oder nur geringfügig beeinflusst. Alternativ kann der Tragegriff 40 auch fest an der Deckplatte 39b angeordnet sein. Mittels eines Tragegriffs 40 ist eine Portabilität des Beatmungsgeräts 100 verbessert.

Wenn das Grundgerät 1 zumindest einen um 180° rotationssymmetrischen Grundriss aufweist wie in Fig. 5a-e dargestellt, kann das Bedienmodul 39a, welches die Benutzerschnittstelle 2 und die Deckplatte 39b aufweist, statt wie in Fig. 15a auch in einer um 180° versetzten Position fest mit dem Grundgerät 1 verbunden sein, wie in Fig. 15b gezeigt, sodass der Anwender je nach bevorzugtem Anwendungsfall (vgl. Fig. 6c, d) die Wahl zwischen einem Beatmungsgerät 100 mit Patientenanschlüssen 4 nach links oder nach rechts hat. Die Konfiguration kann auch bei dieser Gerätevariante werksseitig oder zum Beispiel durch einen Servicetechniker beim Anwender erfolgen.

### BEZUGSZEICHENLISTE

- 1: Grundgerät
- 1a: Griffmulde
- 1b: gewinkelte Anschlagfläche
- 1c: senkrechte Anschlagfläche
- 1d: Nut- und Federelemente
- 1e: kammartige Elemente
- 1f: Sockel
- 2: Benutzerschnittstelle
- 3: Trolley
- 4, 4a, 4b: Patientenanschlüsse
- 5, 5a, 5b: Beatmungsschläuche
- 6: vertikales Drehgelenk
- 7: horizontales Drehgelenk
- 8: Kabel
- 9: Drehmechanismus
- 10: Deckplatte
- 10a: Rampe
- 10b: Vertiefung
- 10c: Vorsprung
- 11: Schwenkgelenk
- 11a: Schwenkgelenk-Gehäuse
- 11b: Gehäuseöffnung
- 11c: Gegenlager
- 12: Patientenbett
- 12a: Kopfende
- 12b: Fußende
- 13: Gasflasche
- 14: Aufnahmeplatte
- 14a: Griff
- 15: Gleitscheibe
- 15a: Kegelfläche
- 15b: äußere Zylinderfläche
- 15c: zentrale Öffnung
- 15d: Anschlagnase
- 16: Federblech
- 16a: Blattfeder
- 17: Halteblech
- 18: Rastnoppe
- 19: Stützrolle
- 20: Anschlagstift
- 21: Gleitlagerring
- 22: Lagerblech
- 23: Gleitstein
- 24: Federscheibe
- 25: erste Kugelraste
- 26: Schraubenkopf
- 27: Durchführung
- 28: Achsstück
- 29: Lagerblock
- 30: Klemmschraube
- 31: Zugfeder
- 32: Haltestruktur
- 33: zweite Kugelraste
- 34: Hülse
- 35: Rastkulisse
- 35a: erste Einbuchtung
- 35b: weitere Einbuchtung
- 35c: Bereich
- 36: Designabdeckung
- 36a: vertikale Achse
- 36b: horizontale Achse
- 36c: Riegelelement
- 36d: Fingermulde
- 36e: Seitenkante
- 36f: Unterkante
- 37: Kabel & Schläuche
- 38: Tischplatte
- 39a: Bedienmodul
- 39b: Deckplatte
- 39c: Mulde
- 40: Tragegriff
- 100: Beatmungsgerät
- h: Hebellänge

## Patentansprüche

1. Beatmungsgerät (100), aufweisend ein Grundgerät (1) mit einer Deckplatte (10), am Grundgerät (1) angeordnete Patientenanschlüsse (4) und eine an der Deckplatte (10) angeordnete Benutzerschnittstelle (2),
wobei die Deckplatte (10) relativ zum Grundgerät (1) um unterschiedliche Winkel in Bezug auf eine vertikale Achse an dem Grundgerät (1) anordenbar ist und
wobei die Benutzerschnittstelle (2) über ein Schwenkgelenk (11) um eine horizontale Schwenkachse verschwenkbar an der Deckplatte (10) gehalten ist, wobei die Benutzerschnittstelle (2) in einer Gebrauchsposition der Deckplatte (10) mittels des Schwenkgelenks (11) in eine Sperrposition verschwenkbar ist,
wobei in der Sperrposition ein relatives Verdrehen der Deckplatte (10) zum Grundgerät (1) gesperrt ist,
wobei das Sperren in der Sperrposition durch die Benutzerschnittstelle (2) erfolgt, welche in der Sperrposition mit dem Grundgerät (1) formschlüssig in Eingriff gerät, und
wobei die Benutzerschnittstelle (2) aus der Sperrposition in eine Freigabeposition verschwenkbar ist, in welcher ein relatives Verdrehen der Deckplatte (10) zum Grundgerät (1) freigegeben ist.

2. Beatmungsgerät (100) nach Anspruch 1,
**dadurch gekennzeichnet, dass** die Deckplatte (10) über einen Drehmechanismus (9) relativ zum Grundgerät (1) um die vertikale Achse verdrehbar an dem Grundgerät (1) gehalten ist.

3. Beatmungsgerät (100) nach Anspruch 2,
**dadurch gekennzeichnet, dass** das Beatmungsgerät (100) einen Rastmechanismus aufweist, welcher ein relatives Verdrehen der Deckplatte (10) zum Grundgerät (1) in vordefinierten Abständen spürbar erschwert.

4. Beatmungsgerät (100) nach Anspruch 2 oder 3,
**dadurch gekennzeichnet, dass** das Beatmungsgerät (100) Drehanschläge zum Begrenzen einer Drehung der Deckplatte (10) um die vertikale Achse relativ zum Grundgerät (1) aufweist.

5. Beatmungsgerät (100) nach einem der vorangehenden Ansprüche,
**dadurch gekennzeichnet, dass** die Deckplatte (10) einen um 180° oder um 90° rotationssymmetrisch ausgebildeten Grundriss aufweist.

6. Beatmungsgerät (100) nach einem der vorangehenden Ansprüche,
**dadurch gekennzeichnet, dass**
die Deckplatte (10) eine Form aufweist, welche einer Grundrissform des Grundgeräts (1) entspricht, sodass die Deckplatte (10) zumindest bei entsprechender Ausrichtung bündig mit Seitenwänden des Grundgeräts (1) abschließt.

7. Beatmungsgerät (100) nach einem der vorangehenden Ansprüche,
**dadurch gekennzeichnet, dass** das Beatmungsgerät (100) eine Rastvorrichtung zum Halten der Benutzerschnittstelle (2) in einer Rastposition relativ zur Deckplatte (10) aufweist.

8. Beatmungsgerät (100) nach einem der vorangehenden Ansprüche,
**dadurch gekennzeichnet, dass** das Schwenkgelenk (11) ausgebildet ist, die Benutzerschnittstelle (2) in eine horizontale Position zu verschwenken.

9. Beatmungsgerät (100) nach einem der vorangehenden Ansprüche,
**dadurch gekennzeichnet, dass** die Sperrposition derart festgelegt ist, dass die Benutzerschnittstelle in einer Bedienposition verschwenkt ist,
wobei die Bedienposition sich über einen Verschwenkbereich zwischen einer vertikalen Ausrichtung der Benutzerschnittstelle bis zu einer Übergangsausrichtung der Benutzerschnittstelle erstreckt,
wobei die Freigabeposition derart definiert ist, dass die Benutzerschnittstelle zwischen der Übergangsausrichtung und der horizontalen Ausrichtung verschwenkt ist.

10. Beatmungsgerät (100) nach einem der vorangehenden Ansprüche,
**dadurch gekennzeichnet, dass** das Schwenkgelenk (11) ausgebildet ist, in der Sperrposition die Deckplatte (10) in der Gebrauchsposition zu sperren.

11. Beatmungsgerät (100) nach einem der vorangehenden Ansprüche,
**dadurch gekennzeichnet, dass** das Grundgerät (1) auf einem Trolley (3) des Beatmungsgeräts (100) angeordnet ist.

12. Beatmungsgerät (100) nach Anspruch 11,
**dadurch gekennzeichnet, dass** das Grundgerät (1) über eine Aufnahmeplatte (14) des Trolleys (3) an dem Trolley (3) lösbar gehalten ist.

13. Beatmungsgerät (100) nach Anspruch 12,
**dadurch gekennzeichnet, dass** das Grundgerät (1) und die Aufnahmeplatte derart ausgebildet sind, dass das Grundgerät (1) in zwei um 180° voneinander verschiedenen Positionen oder in mindestens drei um 90° voneinander verschiedenen Positionen auf der Aufnahmeplatte anordenbar sowie fixierbar ist.

14. Beatmungsgerät (100) nach einem der vorangehenden Ansprüche,
**dadurch gekennzeichnet, dass** die Benutzerschnittstelle (2) linear verschiebbar an der Deckplatte (10) angeordnet ist.

15. Beatmungsgerät (100) nach einem der Ansprüche 2 bis 4,
**dadurch gekennzeichnet, dass** der Drehmechanismus (9) eine zentrale Durchführung (27) aufweist, durch welche Kabel zum elektrischen Koppeln der Benutzerschnittstelle (2) mit dem Grundgerät (1) hindurchführbar oder hindurchgeführt sind.

16. Beatmungsgerät nach einem der vorangehenden Ansprüche, **dadurch gekennzeichnet, dass** alle Geräteanschlüsse (37) hinter einer Abdeckung (36) des Grundkörpers (1) untergebracht sind,
wobei die Abdeckung (36) den Grundriss des Grundgeräts (1) nicht überschreitet.

## Claims

1. Ventilator (100), having a basic device (1) with a cover plate (10), patient connections (4) arranged on the basic device (1), and a user interface (2) arranged on the cover plate (10),
wherein the cover plate (10) can be arranged on the basic device (1) at different angles relative to the basic device (1) with respect to a vertical axis, and
wherein the user interface (2) is held on the cover plate (10) so as to be pivotable via a pivot joint (11) about a horizontal pivot axis,
wherein the user interface (2) is pivotable into a blocking position by means of the pivot joint (11) in a use position of the cover plate (10), wherein, in the blocking position, a relative rotation of the cover plate (10) with respect to the basic device (1) is blocked,
wherein the blocking in the blocking position is undertaken by the user interface (2) which, in the blocking position, enters into engagement in a form-fitting manner with the basic device (1), and
wherein the user interface (2) is pivotable from the blocking position into a release position in which a relative rotation of the cover plate (10) with respect to the basic device (1) is released.

2. Ventilator (100) according to Claim 1,
**characterized in that** the cover plate (10) is held on the basic device (1) via a rotary mechanism (9) so as to be rotatable relative to the basic device (1) about the vertical axis.

3. Ventilator (100) according to Claim 2,
**characterized in that** the ventilator (100) has a latching mechanism which noticeably impedes a relative rotation of the cover plate (10) with respect to the basic device (1) at predefined distances.

4. Ventilator (100) according to Claim 2 or 3,
**characterized in that** the ventilator (100) has rotary stops for limiting a rotation of the cover plate (10) about the vertical axis relative to the basic device (1).

5. Ventilator (100) according to one of the preceding claims,
**characterized in that** the cover plate (10) has a basic contour formed in a rotationally symmetrical manner about 180° or about 90°.

6. Ventilator (100) according to one of the preceding claims,
**characterized in that** the cover plate (10) has a shape which corresponds to a basic contour shape of the basic device (1) such that the cover plate (10), at least when correspondingly aligned, ends flush with side walls of the basic device (1).

7. Ventilator (100) according to one of the preceding claims,
**characterized in that** the ventilator (100) has a latching device for holding the user interface (2) in a latching position relative to the cover plate (10).

8. Ventilator (100) according to one of the preceding claims,
**characterized in that** the pivot joint (11) is designed to pivot the user interface (2) into a horizontal position.

9. Ventilator (100) according to one of the preceding claims,
**characterized in that** the blocking position is defined in such a manner that the user interface is pivoted in an operating position,
wherein the operating position extends over a pivoting range between a vertical alignment of the user interface as far as a transition alignment of the user interface,
wherein the release position is defined in such a manner that the user interface is pivoted between the transition alignment and the horizontal alignment.

10. Ventilator (100) according to one of the preceding claims,
**characterized in that** the pivot joint (11) is designed, in the blocking position, to block the cover plate (10) in the use position.

11. Ventilator (100) according to one of the preceding claims,
**characterized in that** the basic device (1) is arranged on a trolley (3) of the ventilator (100).

12. Ventilator (100) according to Claim 11,
**characterized in that** the basic device (1) is held releasably on the trolley (3) via a receiving plate (14) of the trolley (3).

13. Ventilator (100) according to Claim 12,
**characterized in that** the basic device (1) and the receiving plate are designed in such a manner that the basic device (1) can be arranged and can be fixed on the receiving plate in two positions differing from each other by 180° or in at least three positions differing from one another by 90°.

14. Ventilator (100) according to one of the preceding claims,
**characterized in that** the user interface (2) is arranged in a linearly displaceable manner on the cover plate (10).

15. Ventilator (100) according to one of Claims 2 to 4,
**characterized in that** the rotary mechanism (9) has a central leadthrough (27) through which cables for electrically coupling the user interface (2) to the basic device (1) can be led or are led.

16. Ventilator according to one of the preceding claims,
**characterized in that** all of the device connections (37) are accommodated behind a covering (36) of the basic body (1),
the covering (36) not exceeding the basic contour of the basic device (1).

## Revendications

1. Respirateur (100) comprenant un appareil de base (1) muni d'un panneau de recouvrement (10), des raccords (4) dédiés à des patients et disposés au niveau de l'appareil de base (1), et un interface d'utilisateur (2) situé au niveau dudit panneau de recouvrement (10),
lequel panneau de recouvrement (10) peut être implanté, vis-à-vis de l'appareil de base (1), suivant des angles différents par rapport à un axe vertical sur ledit appareil de base (1),
l'interface d'utilisateur (2) étant retenu au niveau dudit panneau de recouvrement (10) avec faculté de pivotement autour d'un axe horizontal de pivotement, par l'intermédiaire d'une articulation pivotante (11),
ledit interface d'utilisateur (2) étant apte à pivoter vers une position de blocage, au moyen de ladite articulation pivotante (11), lorsque ledit panneau de recouvrement (10) occupe une position d'utilisation,
sachant qu'une rotation relative dudit panneau de recouvrement (10), par rapport à l'appareil de base (1), est bloquée dans ladite position de blocage,
sachant que le blocage s'opère, dans la position de blocage, sous l'action dudit interface d'utilisateur (2) venant en prise avec l'appareil de base (1) par complémentarité de formes, dans ladite position de blocage,
lequel interface d'utilisateur (2) peut pivoter, à partir de ladite position de blocage, vers une position de libération dans laquelle une rotation relative du panneau de recouvrement (10) est autorisée par rapport à l'appareil de base (1).

2. Respirateur (100) selon la revendication 1,
**caractérisé par le fait que** le panneau de recouvrement (10) est retenu vis-à-vis de l'appareil de base (1), par l'intermédiaire d'un mécanisme rotatif (9), avec faculté de rotation autour de l'axe vertical sur ledit appareil de base (1).

3. Respirateur (100) selon la revendication 2,
**caractérisé par le fait que** ledit respirateur (100) est pourvu d'un mécanisme d'encliquetage qui, à intervalles prédéfinis, rend perceptiblement plus difficile une rotation relative du panneau de recouvrement (10) par rapport à l'appareil de base (1).

4. Respirateur (100) selon la revendication 2 ou 3,
**caractérisé par le fait que** ledit respirateur (100) est doté de butées rotatoires conçues pour limiter une rotation du panneau de recouvrement (10) par rapport à l'appareil de base (1), autour de l'axe vertical.

5. Respirateur (100) selon l'une des revendications précédentes, **caractérisé par le fait que** le panneau de recouvrement (10) offre un profil de base réalisé avec symétrie de rotation de 180° ou de 90°.

6. Respirateur (100) selon l'une des revendications précédentes, **caractérisé par le fait que** le panneau de recouvrement (10) présente une forme correspondant à une configuration de base de l'appareil de base (1), de façon telle que ledit panneau de recouvrement (10) se trouve dans l'affleurement de parois latérales dudit appareil de base (1) moyennant au moins une orientation correspondante.

7. Respirateur (100) selon l'une des revendications précédentes, **caractérisé par le fait que** ledit respirateur (100) est muni d'un dispositif d'encliquetage dévolu à la retenue de l'interface d'utilisateur (2) par rapport au panneau de recouvrement (10) dans une position d'encliquetage.

8. Respirateur (100) selon l'une des revendications précédentes, **caractérisé par le fait que** l'articulation pivotante (11) est conçue pour faire pivoter l'interface d'utilisateur (2) vers une position horizontale.

9. Respirateur (100) selon l'une des revendications précédentes, **caractérisé par le fait que** la position de blocage et fermement établie de façon telle que l'interface d'utilisateur (2) soit animé de pivotements, dans une position de service,
laquelle position de service s'étend sur une plage de pivotements, entre une orientation verticale de l'interface d'utilisateur et une orientation transitoire dudit interface d'utilisateur,
la position de libération étant bien définie, de telle sorte que des pivotements soient imprimés audit interface d'utilisateur entre ladite orientation transitoire et l'orientation horizontale.

10. Respirateur (100) selon l'une des revendications précédentes, **caractérisé par le fait que**, dans la position de blocage, l'articulation pivotante (11) est conçue pour bloquer le panneau de recouvrement (10) dans la position d'utilisation.

11. Respirateur (100) selon l'une des revendications précédentes, **caractérisé par le fait que** l'appareil de base (1) est disposé sur un chariot (3) dudit respirateur (100).

12. Respirateur (100) selon la revendication 11,
**caractérisé par le fait que** l'appareil de base (1) est retenu amoviblement, au niveau du chariot (3), par l'intermédiaire d'une platine réceptrice (14) dudit chariot (3).

13. Respirateur (100) selon la revendication 12,
**caractérisé par le fait que** l'appareil de base (1) et la platine réceptrice sont conçus de façon telle que ledit appareil de base (1) puisse être tant implanté, que consigné à demeure, sur ladite platine réceptrice, dans deux positions différant l'une de l'autre de 180°, ou dans au moins trois positions différant de 90° les unes des autres.

14. Respirateur (100) selon l'une des revendications précédentes, **caractérisé par le fait que** l'interface d'utilisateur (2) est agencé avec faculté de déplacement linéaire au niveau du panneau de recouvrement (10).

15. Respirateur (100) selon l'une des revendications 2 à 4,
**caractérisé par le fait que** le mécanisme rotatif (9) est muni d'une traversée centrale (27) franchie, ou pouvant être franchie par des câbles affectés au couplage électrique de l'interface d'utilisateur (2) avec l'appareil de base (1).

16. Respirateur (100) selon l'une des revendications précédentes, **caractérisé par le fait que** toutes les connexions (37) de l'appareil sont logées derrière un capot (36) du corps de base (1),
lequel capot (36) ne dépasse pas au-delà du profil de base de l'appareil de base (1).
